# EUROPEAN PATENT SPECIFICATION

(11) **EP 3 258 969 B1**
(45) Date of publication and mention of the grant of the patent: **20.03.2019**
(21) Application number: 16705139.0
(22) Date of filing: 17.02.2016
(51) Int. Cl.: A61K 47/50

(54) **IMMUNOCONJUGATES FOR SPECIFIC INDUCTION OF T CELL CYTOTOXICITY AGAINST A TARGET CELL**
IMMUNKONJUGATE FÜR DIE SPEZIFISCHE INDUKTION VON T-ZELLZYTOTOXIZITÄT GEGEN EINE ZIELZELLE
IMMUNOCONJUGUÉS POUR L'INDUCTION SPÉCIFIQUE DE CYTOTOXICITÉ DES LYMPHOCYTES T CONTRE UNE CELLULE CIBLE

(30) Priority: 18.02.2015 EP 15155598; 10.12.2015 EP 15199306
(43) Date of publication of application: 27.12.2017
(73) Proprietor: F. Hoffmann-La Roche AG, 4070 Basel (CH)
(72) Inventor: DZIADEK, Sebastian, 83671 Benediktbeuern (DE); LIFKE, Alexander, 82377 Penzberg (DE); LIFKE, Valeria, 82377 Penzberg (DE); HILLRINGHAUS, Lars, 82549 Königsdorf-Schönrain (DE)
(74) Representative: Burger, Alexander
(86) International application number: PCT/EP2016/053332
(87) International publication number: WO 2016/131856

(56) References cited:
- WO-A1-00/12706
- WO-A1-99/45954
- WO-A1-2014/096015
- WO-A2-2005/066203
- WO-A2-2009/051555
- DATABASE BIOSIS, [Online] 16 November 2007 (2007-11-16), HARO KURTIS J ET AL: "Photo-reactive and non-natural amino acid Epitopes of human WT1 enhance immunogenicity and allow kinetic study of antigen processing", XP002670071, retrieved from BIOSIS Database accession no. PREV200800217583
- T. Storni ET AL: "Loading of MHC Class I and II Presentation Pathways by Exogenous Antigens: A Quantitative In Vivo Comparison", THE JOURNAL OF IMMUNOLOGY, vol. 172, no. 10, 5 May 2004 (2004-05-05), pages 6129-6135, XP055259622, US ISSN: 0022-1767, DOI: 10.4049/jimmunol.172.10.6129

## Description

### FIELD OF THE INVENTION

The present invention relates to immunoconjugates for specific induction of T cell cytotoxicity against a target cell, comprising at least one T cell response eliciting peptide that is presentable via MHC class I coupled to a target cell binding moiety and methods of their production and uses thereof.

### BACKGROUND OF THE INVENTION

T cells, in contrast to B cells, recognize peptides derived from processed antigens, which are presented on the cell surface via autologous cell surface molecules of the major histocompatibility complex (MHC).

Current thinking of antigen presentation via MHC class II (MHC II) is that exogenous proteins access MHC II through phagocytosis and endocytosis, and endogenous proteins access the MHC II through autophagy. Antigens are processed to peptide antigens (peptide epitopes) of an appropriate length (∼10-16 amino acids) for binding to MHC II molecules in the phagosome, endosome or autophagosome. Unloaded MHC II αβ-chain dimers are assembled in the endoplasmic reticulum (ER) as a nonameric complex with the invariant chain (Ii), which protects against premature peptide or protein interactions in pre-lysosomal compartments. This complex traffics to the lysosomal MHC II compartment, where the invariant chain is subjected to sequential proteolysis. The final cleavage product, a peptide known as class II-associated Ii peptide (CLIP), occupies the peptide-binding groove and must be released prior to loading MHC II molecules with high-affinity peptides (MHC II restricted peptides) (Wearsch P and Creswell P, Nat. Rev. Immunol.: Antigen processing and presentation, Poster available online, http://www.nature.com/nri/posters/antigenprocessing).

Binding of naive CD4 T cells to peptide-loaded MHC II on the cell surface mediates immunization to the antigen by priming the naive CD4 T cell to polarize into effector T cells (Th1, Th2, Th17) or memory T cells. Alternatively depending on the local milieu (e.g. cytokine balance) in the microenvironment of the APC, binding of naive CD4 T cells to peptide-loaded MHC II may mediate immune tolerance to an antigen by priming the naive CD4 T cell to polarize into regulatory T cells (Treg).

While MHC II molecules normally occur only on antigen presenting cells, like macrophages, B cells and dendritic cells, MHC class I (MHC I) occurs on all nucleated cells.

The currently postulated mechanism of antigen presentation via MHC class I (MHC I) is that endogenous antigens, such as viral proteins or autologous proteins that are produced inside the cell, are processed into peptides inside the cytosol by the proteasome and cytosolic proteases. These peptides are then transported into the ER through specific pumps called the transporter associated with antigen processing (TAP) complex, where they are further trimmed by ER aminopeptidases (ERAPs) to produce peptides of 8-12 amino acids.

It has been demonstrated that peptide epitopes of exogenous proteins that are internalized into cells, preferentially into professional antigen presenting cells (APCs) like dendritic cells or macrophages, can also be presented by MHC I. This is attributed to the presence of a translocation domain comprised in the exogenous protein that supports retrotranslocation of said protein from a phagosome or endosome and thereby directs the proteins into the cytoplasm of the cell (Donnelly JJ et al. Proc Natl Acad Sci U S A. 1993 Apr 15;90(8):3530-4; Wearsch P and Creswell P, Nat. Rev. Immunol.: Antigen processing and presentation, Poster available online, http://www.nature.com/nri/posters/antigenprocessing).

The assembly of MHC I heavy chain-β2-microglobulin heterodimers with the short peptides is coordinated by the peptide-loading complex, which is composed of a disulphide-linked dimer of tapasin and ERp57, calreticulin (CRT) and TAP molecules. Tapasin also supports peptide editing to select for the presentation of stable peptide-MHC I complexes on the cell surface (Wearsch P and Creswell P, Nat. Rev. Immunol.: Antigen processing and presentation, Poster available online, http://www.nature.com/nri/posters/antigenprocessing). Once the peptide is loaded onto the MHC I molecule, the peptide-loading complex dissociates and the peptide-loaded MHC I leaves the ER through the secretory pathway to reach the cell surface. Peptides that fail to bind MHC I molecules in the lumen of the ER are removed from the ER via the sec61 channel into the cytosol, where they might undergo further trimming in size, and might be translocated by TAP back into ER for binding to an MHC I molecule (Koopmann JO, Albring J, Hüter E, et al. (July 2000) Immunity, 13 (1): 117-27.; Albring J, Koopmann JO, Hämmerling GJ, Momburg F (January 2004), Mol. Immunol. 40 (10): 733-41).

MHC I molecules on the cell surface present their loaded peptides to cytotoxic CD8 T lymphocytes (CD8 T cells, also called CTLs). CTLs trigger the cell, which is presenting peptide epitopes via MHC I, to undergo programmed cell death by apoptosis. Hence, MHC I mediates cell-mediated immunity, which is the immune systems' primary means to address intracellular pathogens but also to address self cells expressing mutated or abnormal proteins.

It has been a focus of research during the past years, to exploit the mechanism of inducing cell-mediated immunity in order to induce killing of a variety of diseased cells (e.g. tumor cells) that are not affected by intracellular pathogens. As all nucleated cells express MHC I, inducing presentation of antigens of intracellular pathogens via MHC I is a promising approach to induce programmed cell death via CTLs on a variety of diseased cell populations, e.g. tumor cells.

In this regard, approaches have been developed to deliver antigens derived from intracellular pathogens, e.g. from viruses, by immunoconjugates built up of a binding partner that binds to a target cell and a peptidic fraction of the intracellular pathogen to a certain diseased cell population to induce presentation of processed fragments of the delivered peptides on the surface of the diseased cell via MHC I. The diseased cell will then be eliminated by CTLs.

One approach is disclosed in EP 0659439 A1. EP 0659439 A1 discloses an immunoconjugate, wherein a virus derived peptide (modified influenza matrix peptide 57-68) or a bacterium derived peptide (Plasmodium bergei peptide 249-260) is coupled to a target cell binding antibody. The respective peptide is coupled to the antibody by addition of an N-terminal cysteine residue and subsequent crosslinking via a SPDP-linker to the antibody to produce the immunoconjugate. Upon internalization the respective peptide including the added N-terminal cysteine residue is split off from the conjugate, further processed within the cell and presented by MHC I. However, the IC50 value of the immunoconjugates was very high (0,5µmol/l).

As described in the approach according to EP 0659439 A1, a major challenge is to stimulate an MHC I restricted CTL response against exogenous antigens. In most cases when antigen is provided exogenously, it is not routed to the cytoplasm from where it could be transported to the MHC I pathway.

One methodology to improve the IC50 value for immunoconjugates including peptides that can be presented via MHC I upon processing is suggested in EP 0659439 A1. Here, it is recommended to include a translocation domain in the immunoconjugate in order to rout the immunoconjugate to the cytoplasm via retrotranslocation. However EP 0659439 A1 does not demonstrate results regarding this methodology.

Therefore there is still a need for improved approaches to induce the killing of target cells by inducing cell-mediated immunity.

### SUMMARY OF THE INVENTION

The present invention relates to an immunoconjugate comprising comprising at least one naturally occurring T cell response eliciting peptide that is presentable via MHC class I coupled to a target cell binding antibody via a cleavable bond, wherein the cleavable bond is arranged such that upon cleavage said T cell response eliciting peptide, which is directly presentable via a MHC class I molecule, is released from the immunoconjugate, characterized in that the naturally occurring T cell response eliciting peptide comprises at least one cysteine residue and in that the unmodified T cell response eliciting peptide is covalently coupled to the target cell binding antibody via a disulfide bond.

One embodiment of the invention relates to an immunoconjugate, wherein the cleavable bond is cleavable within the endosomal compartment of the target cell.

One embodiment of the invention relates to an immunoconjugate, wherein the immunoconjugate does not comprise a translocation domain.

One embodiment of the invention relates to an immunoconjugate, wherein in less than 12 hours after internalization of the immunoconjugate into the target cell the T cell response eliciting peptide is presented on the surface of the target cell via an MHC class I molecule.

Another aspect of the invention is a pharmaceutical composition comprising an immunoconjugate according to the invention in combination with a pharmaceutically acceptable carrier.

Another aspect of the disclosure is the use of an immunoconjugate according to the invention for specifically inducing T cell cytotoxicity against the target cell.

Another aspect of the invention is a method for the generation of an immunoconjugate according to the invention, comprising the steps of
a) providing the recombinant target cell binding moiety,
b) providing the T cell response eliciting peptide, and
c) coupling of at least one of said T cell response eliciting peptide to said target cell binding moiety via the cleavable bond.

One embodiment of the invention relates to a method for the generation of an immunoconjugate, wherein an unmodified T cell response eliciting peptide is coupled to the target cell binding moiety.

Another aspect of the invention is the immunoconjugate according to the invention for use as a medicament.

Another aspect of the invention is the immunoconjugate according to the invention comprising a cancer cell binding moiety, for the treatment of cancer.

Another aspect of the invention is the immunoconjugate according to the invention for use in the treatment of infectious diseases, autoimmune diseases, diabetes or allergy.

Another aspect of the disclosure is a method of treatment of a patient suffering from a disease by administering an immunoconjugate according to the invention to the patient in the need of such treatment.

According to the invention the T cell response eliciting peptide cleaved from the immunoconjugate can be directly presented via MHC class I molecules without further processing or trimming within the target cell. The T cell response eliciting peptide is cleaved from the immunoconjugate within the endosomal compartment of the target cell upon internalization of the immunoconjugate, and the peptide is directly (i.e. without further processing or trimming) loaded onto MHC class I molecules in the endosomal compartment and transported to the target cell surface for presentation via MHC class I. Surprisingly, considerably lower IC50 values are reached as compared to approaches known in the art.

### DESCRIPTION OF THE FIGURES

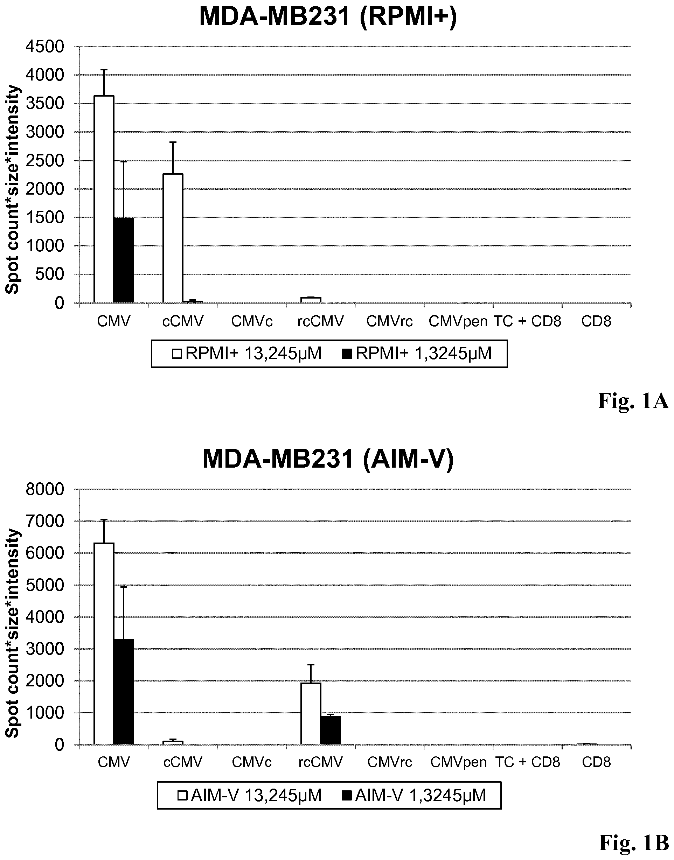

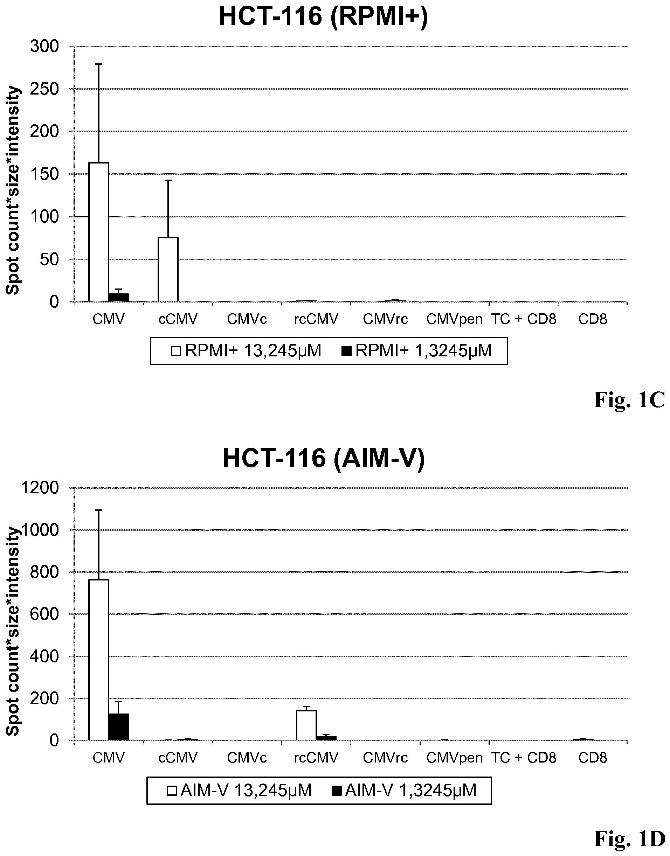

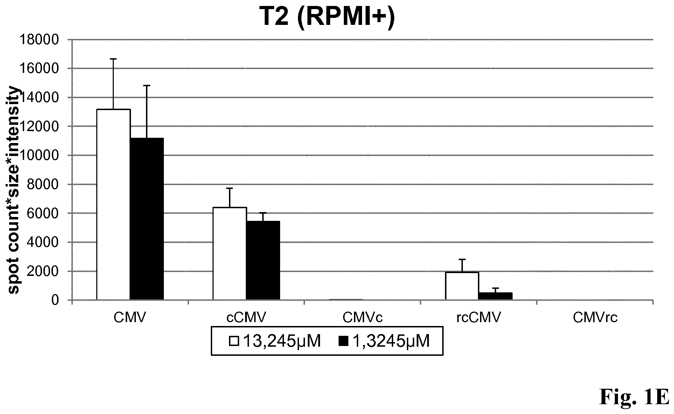

**Figure 1****:** Diminished T cell activation in modified CMV peptides as measured by IFNγ-ELISPOT using different tumor cell lines and cell culture media. **A)** MDA-MB231 cells in RPMI+, **B)** MDA-MB231 cells in AIM-V, **C)** HCT-116 cells in RPMI+, **D)** HCT-116 cells in AIM-V, **E)** T2 cells in RPMI+. Analyses were performed using peptide concentrations of 13,245 µM or 1,3245 µM as indicated.

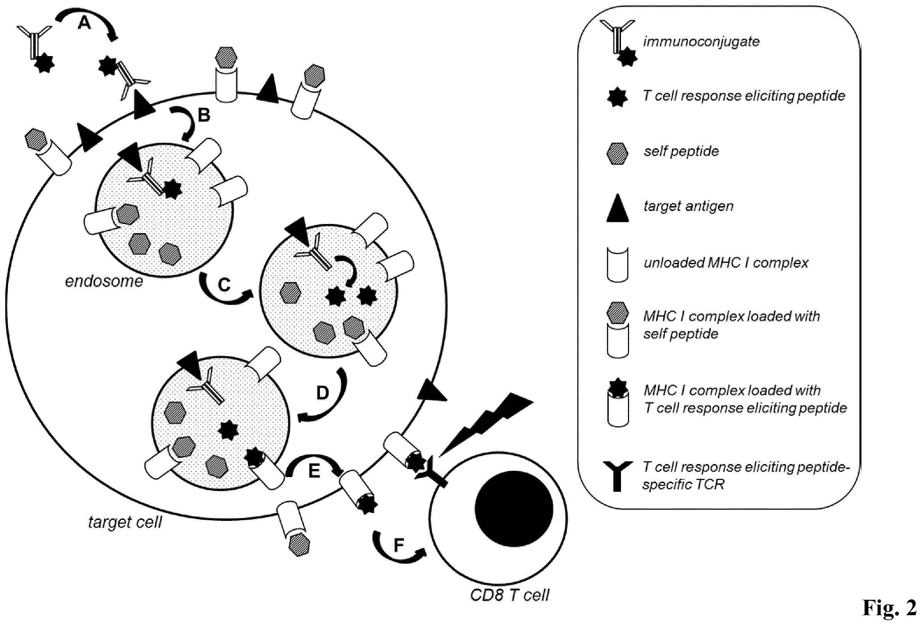

**Figure 2****:** Proposed mode of action of immunoconjugate according to the invention. **A:** Binding of immunoconjugate to target cell. **B:** Internalization of immunoconjugate into endosomal compartment. **C:** Release of T cell response eliciting peptide from the immunoconjugate in the endosomal compartment. **D:** Loading of MHC I molecules with released peptide in endosomal compartment. **E:** Routing of peptide-loaded MHC I molecules to target cell surface. **F:** Recognition of peptide-loaded MHC I molecules on target cell surface by peptide specific CD8 T cells inducing cell death in the target cell.

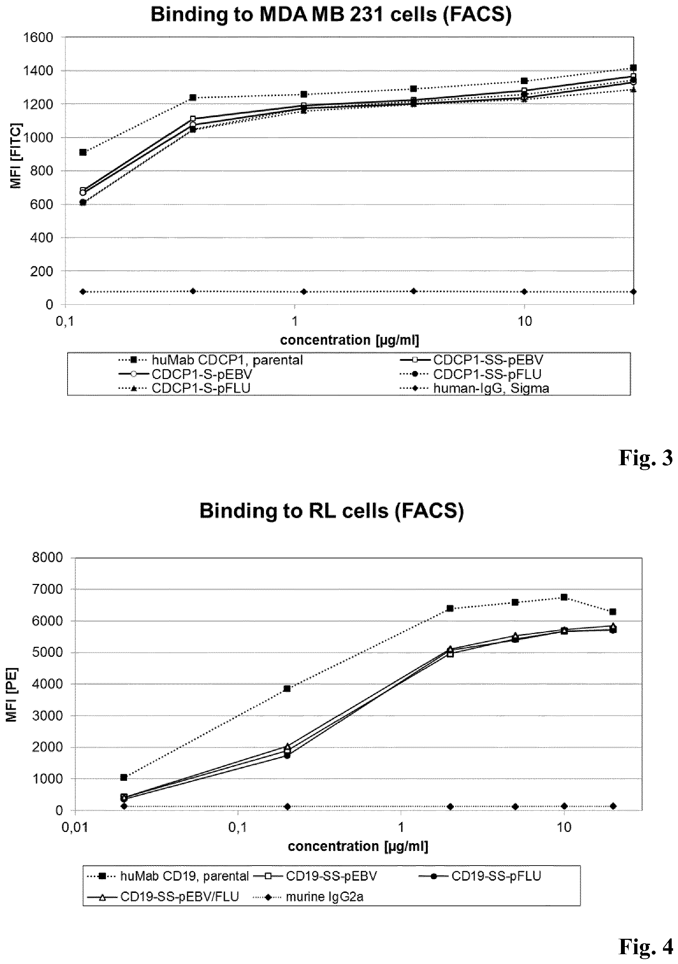

**Figure 3****:** Binding of immunoconjugates comprising anti-CDCPl antibody coupled to EBV and/or FLU peptide to CDCP1 expressing MDA-MB 231 cells.
**Figure 4****:** Binding of immunoconjugates comprising anti-CD19 antibody coupled to EBV and/or FLU peptide to CD19 expressing RL cells.

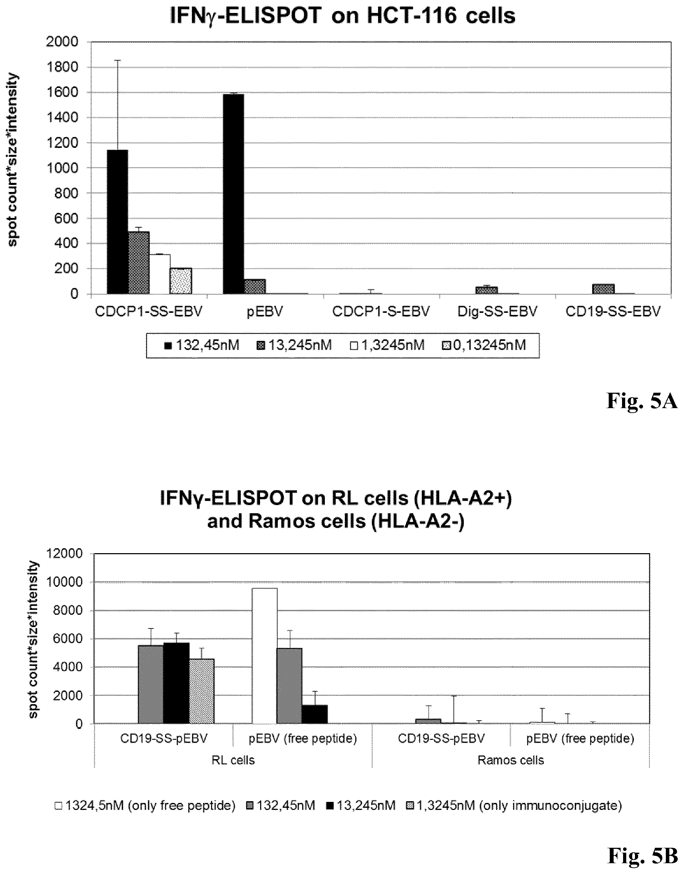

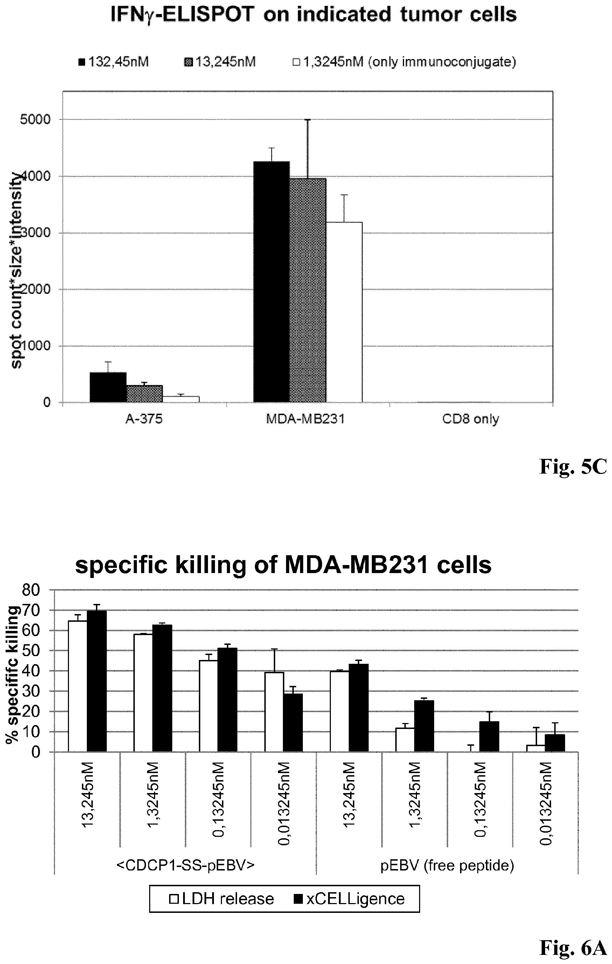

**Figure 5****:** Results of IFN-γ ELISPOT using immunoconjugates according to the invention for peptide-specific T cell activation. Indicated concentrations refer to peptide concentrations. **A)** CDCP-1-expressing HCT-116 cells treated with immunoconjugates comprising monoclonal anti-CDCP-1 antibody and HLA-A2 restricted EBV peptide. **B)** CD19-expressing HLA-A2-positive RL and HLA-A2-negative Ramos cells treated with immunoconjugates comprising monoclonal anti-CD19 antibody and HLA-A2 restricted EBV peptide. **C)** Different CDCP-1-expressing tumor cells treated with immunoconjugates comprising monoclonal anti-CDCP-1 antibody and HLA-A2 restricted EBV peptide.

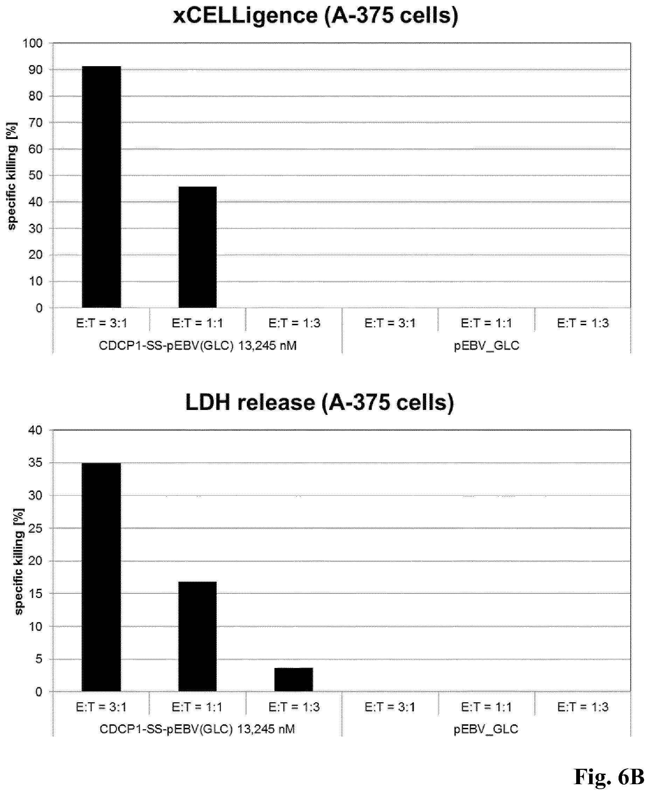

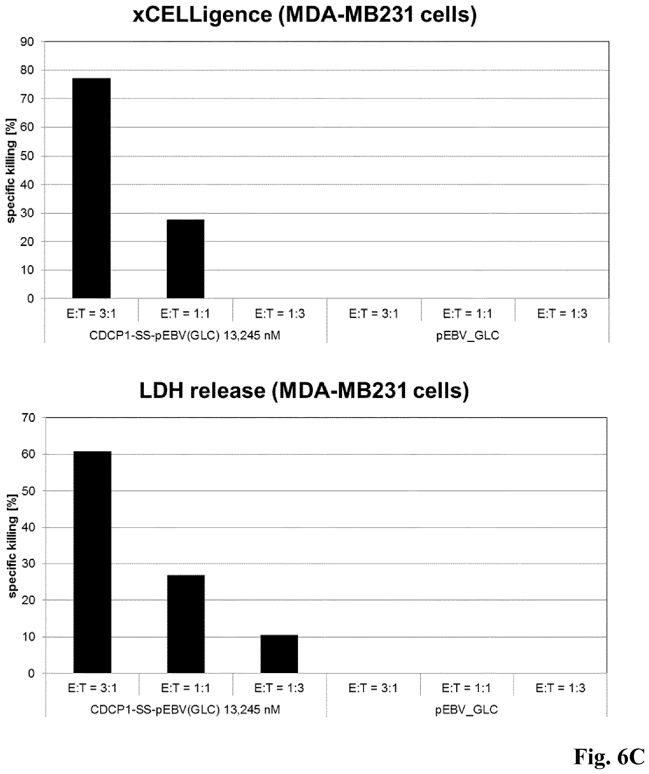

**Figure 6****:** Tumor cell killing of indicated tumor cells assessed by xCELLigence and LDH release assay. **(A)** Tumor cell killing of HLA-A2-positive MDA-MB231 cells mediated by immunoconjugate comprising anti-CDCPl antibody coupled to HLA-A2 restricted EBV-peptide. **(B)** Specific killing of A-375 cells mediated by immunoconjugate comprising anti-CDCPl antibody coupled to HLA-A2 restricted EBV-peptide with a non-terminal cysteine residue. **(C)** Specific killing of HLA-A2-positive MDA-MB231 cells mediated by immunoconjugate comprising anti-CDCPl antibody coupled to HLA-A2 restricted EBV-peptide with a non-terminal cysteine residue. Indicated concentrations refer to peptide concentrations.

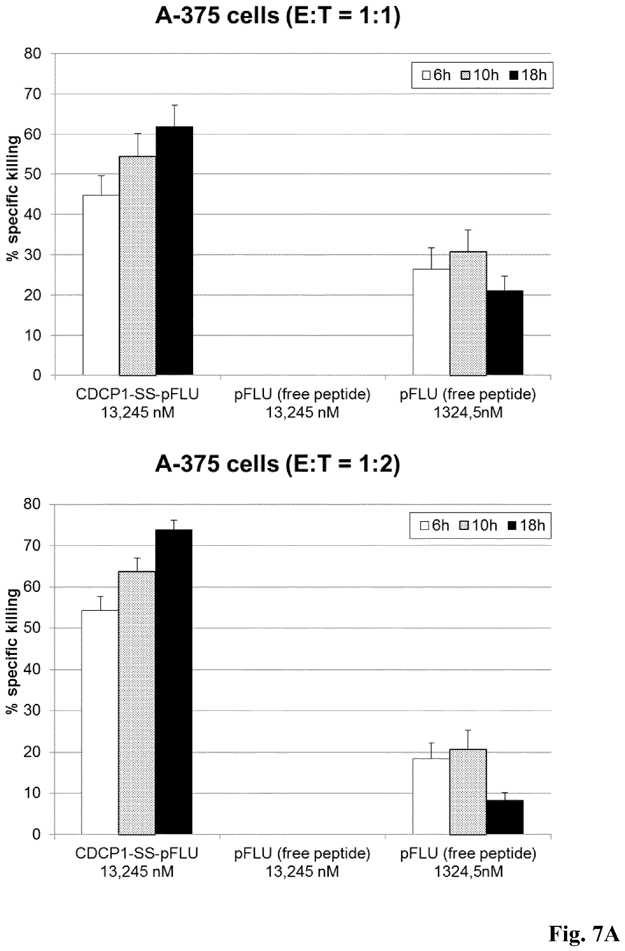

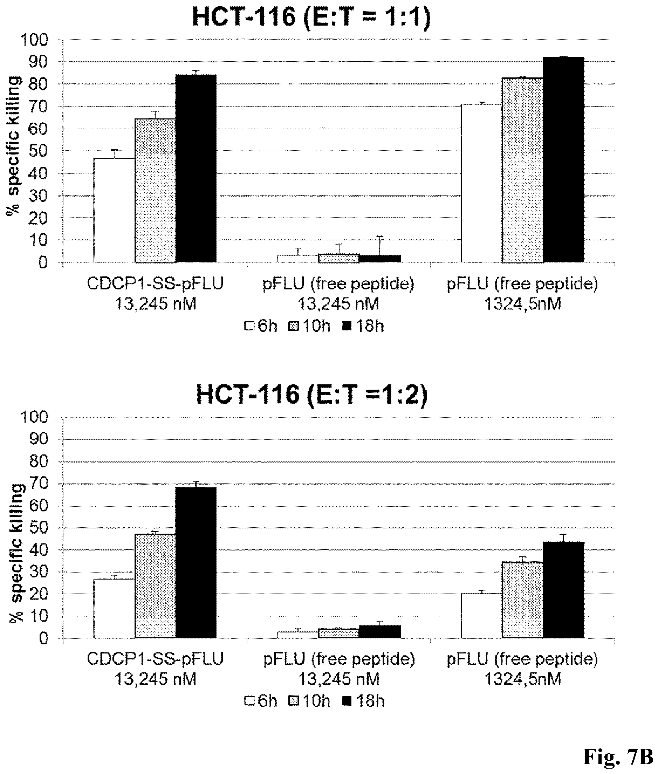

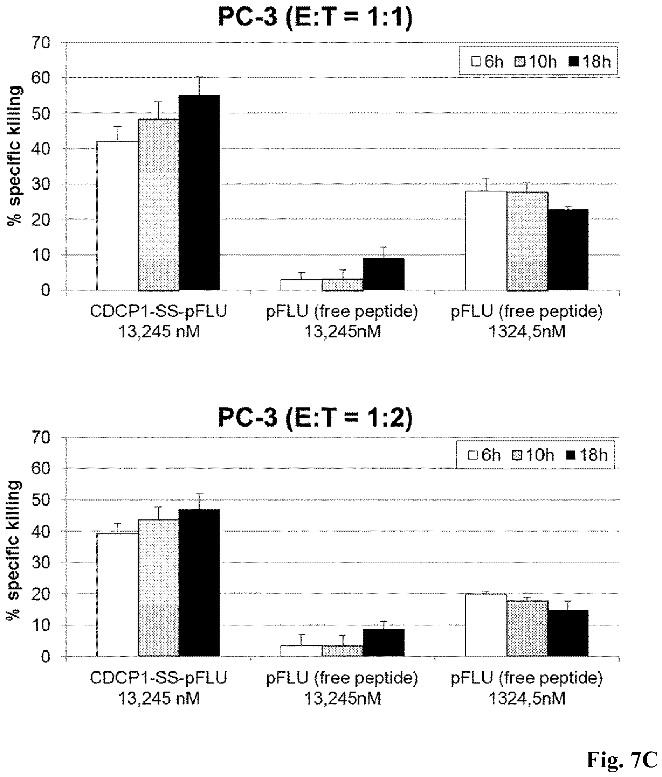

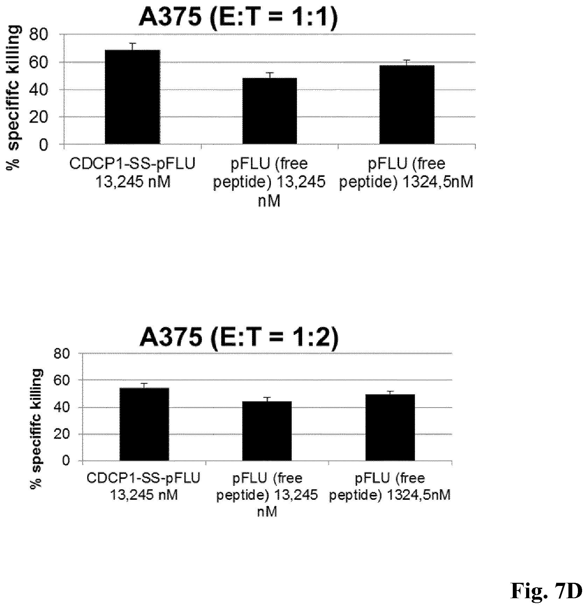

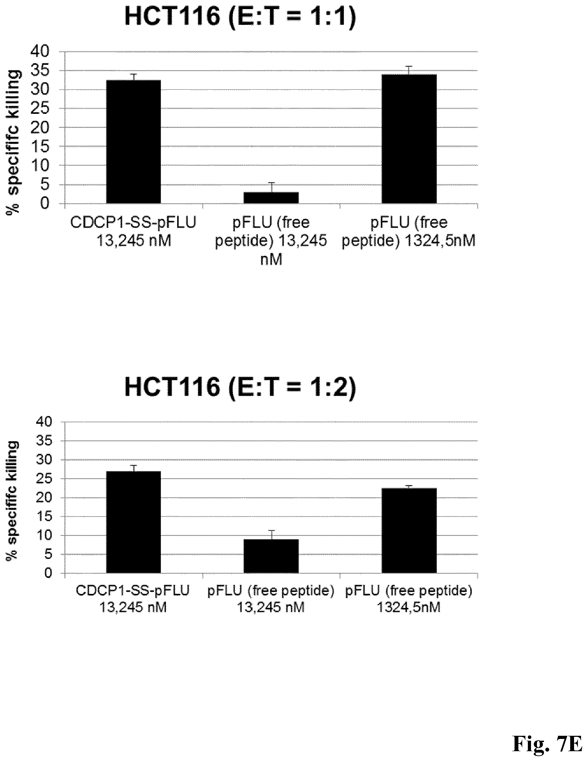

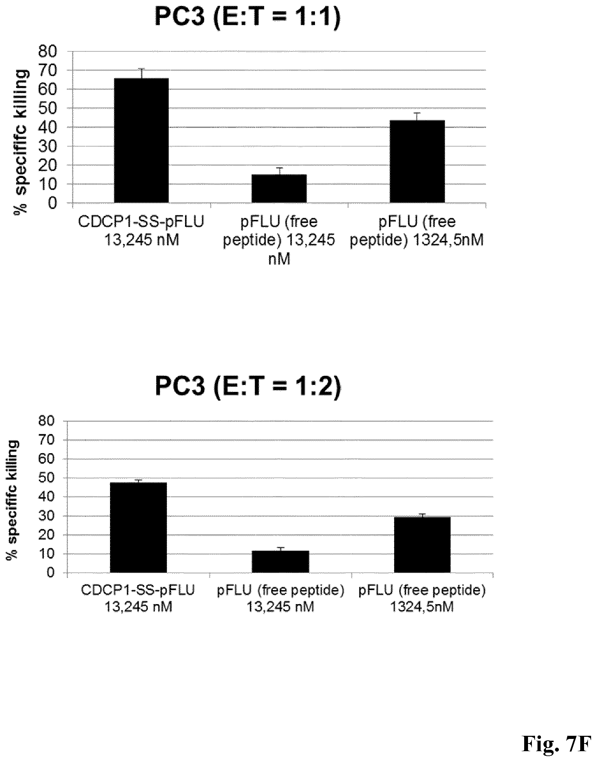

**Figure 7****:** Tumor cell killing of HLA-A1-positive tumor cells mediated by immunoconjugate comprising HLA-A1 restricted FLU-peptide. **A-C)** Results measured by xCELLigence assay after 6, 10 and 18 hours. **D-F)** Results measured by LDH release assay. **A and D)** A-375 cells in effector cell to target cell ratio (E:T) of 1:1 and 1:2, **B and E)** HCT-116 cells, **C and F)** PC3 cells.

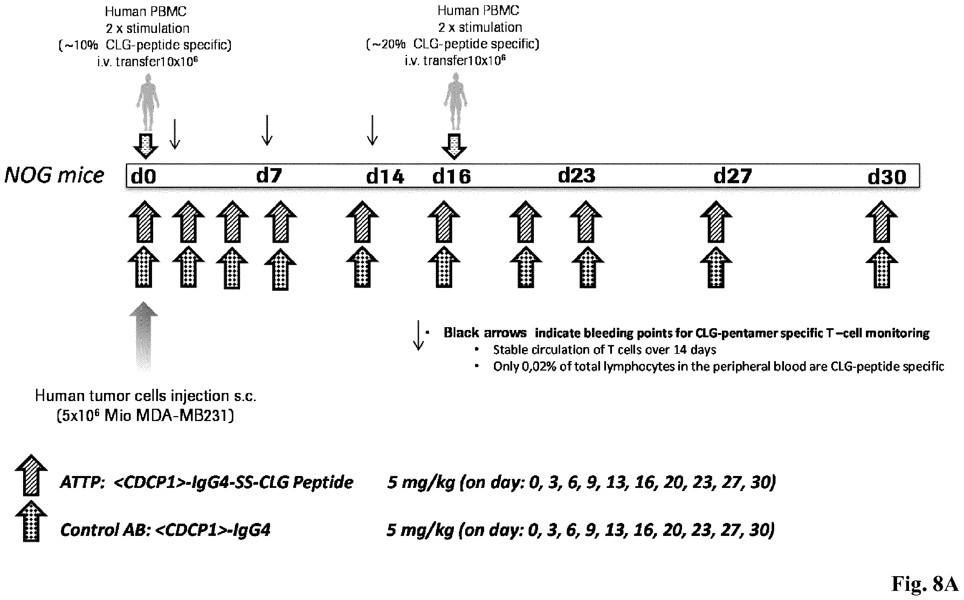

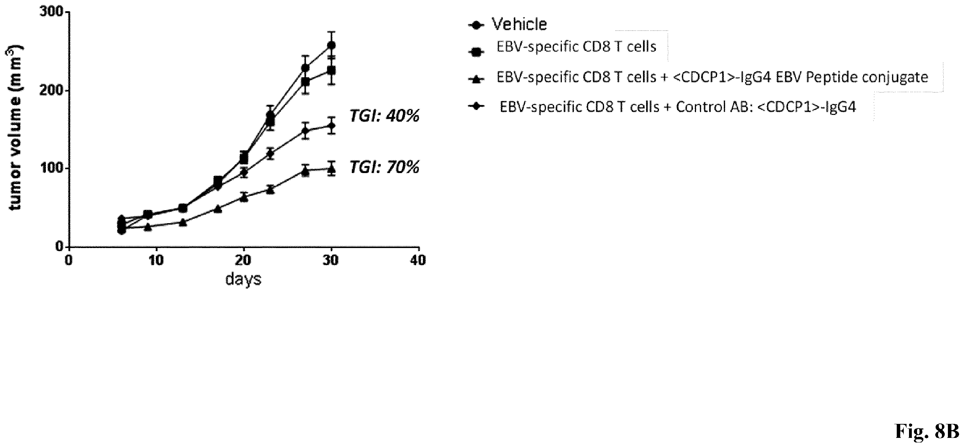

**Figure 8****:** Anti-tumor activity of immunoconjugates according to the invention comprising monoclonal anti-CDCPl antibody in MDA-MB231 s.c. model with i.v. transfer of huPBMCs. Testing scheme (**Fig 8A**) and results (**Fig 8B**).

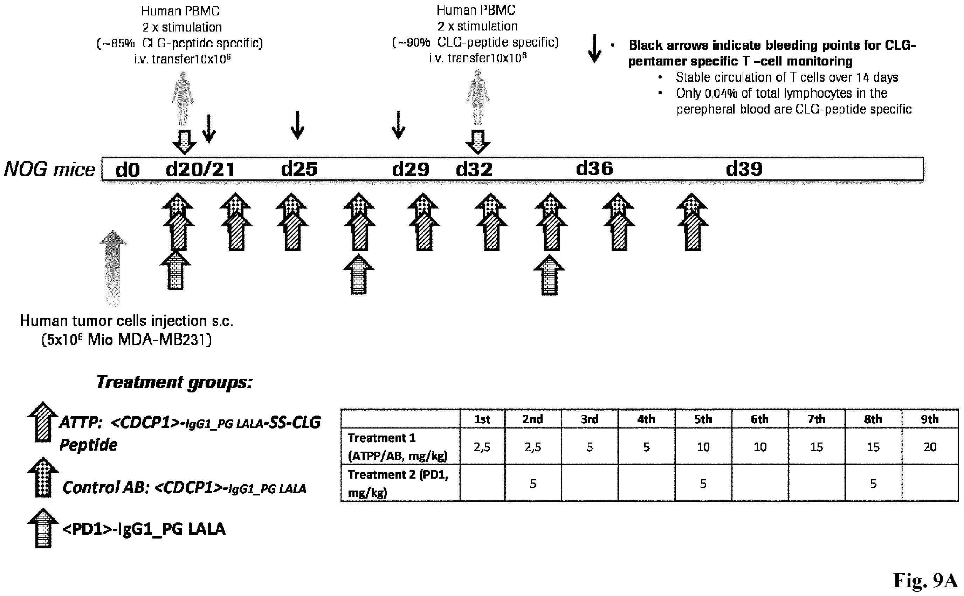

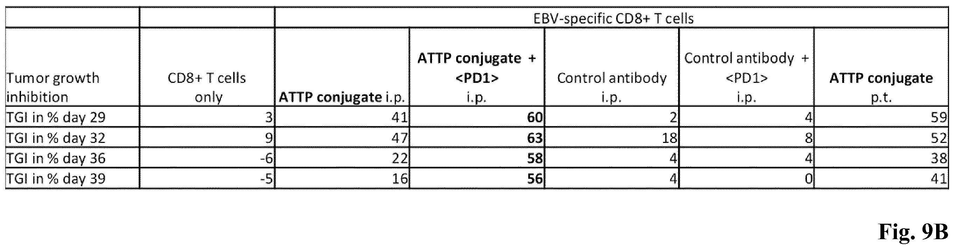

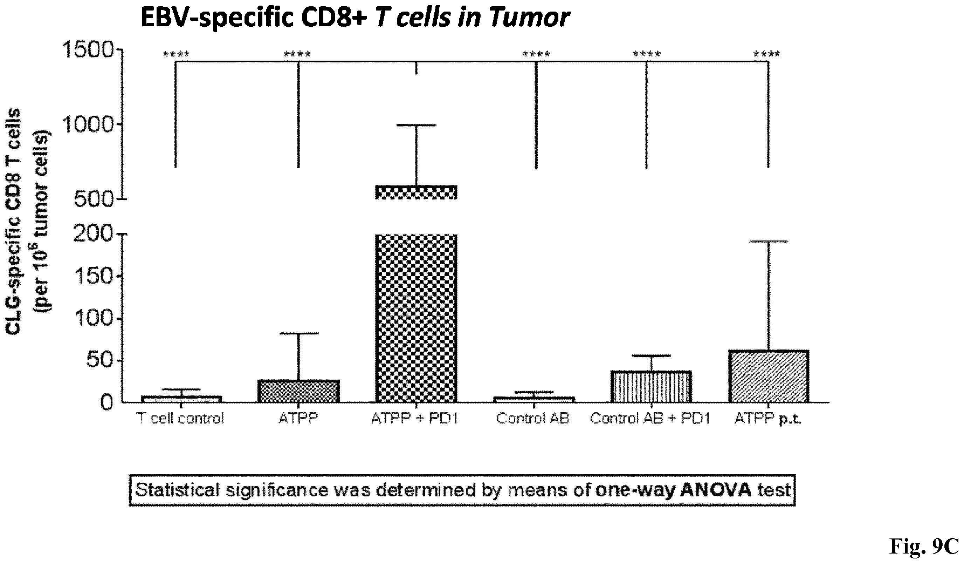

**Figure 9****:** Anti-tumor activity of immunoconjugates according to the invention comprising monoclonal anti-CDCPl antibody in combination with anti-PD1 treatment in established MDA-MB231 s.c. model with i.v. transfer of huPBMCs. Testing scheme (**Fig 9A**) and results of tumor growth inhibition (**Fig 9B**) and T cell increase (**Fig. 9C**).

### DETAILED DESCRIPTION OF THE INVENTION

### 1. Definitions

The term "immunoconjugate" as used herein refers to a target cell binding moiety (e.g. an antibody specifically binding to a target cell or a ligand binding to its receptor on the target cell) coupled to a heterologous functional molecule. The functional molecule essentially present in immunoconjugates according to the invention is a T cell response eliciting peptide. Additionally, further functional molecules, like cytotoxic agents, may be employed.

A "target cell binding moiety" as used herein refers to a moiety that specifically binds to a target cell. The term includes antibodies as well as other natural (e.g. receptors, ligands) or synthetic (e.g. DARPins) molecules capable of specifically binding to a target cell. With "specifically binding to a target cell" as used herein is meant, that said moiety preferentially binds to the target cell within a complex mixture.

The affinity of the binding of an target cell binding moiety to the target cell is defined by the terms kₐ (rate constant for the association of the protein from the protein/target cell complex), k_{D} (dissociation constant), and K_{D} (k_{D}/kₐ). In one embodiment wherein the target cell binding moiety is an antibody specifically binding to a target cell means a binding affinity (K_{D}) of 10⁻⁸ mol/l or less, in one embodiment 10⁻⁸ M to 10⁻¹³ mol/l.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

The term "ligand" herein is used for a molecule, preferably a protein, that forms a complex with a biomolecule on the target cell to serve a biological purpose. In one embodiment, the ligand is a selective ligand that has a tendency to bind to limited types of receptors.

A "peptide" as used herein is a short chain of amino acid monomers linked by peptide bonds. Peptides that bind to MHC class I are typically 8 to 12 amino acids in length. The term "T cell response eliciting peptide that is presentable via MHC class I" as used herein refers to peptides that are presentable via MHC class I molecules but that are not presentable via MHC II and that trigger a CD 8 T cell response. For simplification the term may herein also be referred to as "T cell response eliciting peptide" only.

The T cell response eliciting peptides used within the scope of the invention are typically derived from a non-self (i.e. non-human) pathogenic source. The term "derived from" in this context means that the T cell response eliciting peptide is identical to a naturally occurring T cell epitope of the non-self pathogenic source (i.e. shares the same amino acid sequence). In other words, the amino acid sequence of the T cell response eliciting peptide is identical to a partial sequence of the amino acid sequence of a protein antigen of the non-self pathogenic source. While the pathogenic source of the T cell response eliciting peptide may be of viral or bacterial origin, typically, MHC I presentable T cell response eliciting peptides are derived from intracellular or extracellular pathogens. Intracellular pathogens include viruses, intracellular bacteria or parasites. Extracellular pathogens include parasites and bacteria. Although the T cell response eliciting peptides used for the invention share the same amino acid sequence as the corresponding naturally occurring T cell epitope, the T cell response eliciting peptides of an immunoconjugate according to the invention are typically of synthetic origin.

In the art, peptides derived from intracellular pathogens used for inducing T cell cytotoxicity directed against target cells were occasionally modified by amino acid substitutions or additions in order to improve their physiological properties, e.g. their solubility. For the present invention however only naturally occurring T cell response eliciting peptides are used (i.e. peptides sharing the amino acid sequence of a wild type pathogenic peptide without including further amino acid sequence modifications).

By "naturally occurring" as used herein is meant that the T cell response eliciting peptide that is presentable via MHC class I that is coupled to the target cell binding moiety shares (i.e. consists of) the amino acid sequence of a corresponding non-self pathogenic T cell epitope presentable via MHC class I. In other words, the T cell respopnse eliciting peptide consists of an amino acid sequence that is identical to an amino acid sequence producible by cleavage of the corresponding naturally occurring non-self pathogen within the target cell. Hence, a "naturally occurring T cell response eliciting peptide" or "naturally occurring T cell response eliciting peptide that is presentable via MHC class I" within the terms of the invention may be of synthetic origin, however its amino acid sequence does not comprise any amino acid modifications, in particular no amino acid additions, deletions or substitutions, as compared to a peptide producible by cleavage of the corresponding naturally occurring non-self pathogen. Once again phrased in other words, the amino acid sequence of a naturally occurring T cell response eliciting peptide coupled to a target cell binding moiety is identical to a (in one preferred embodiment 8 to 12 amino acids long) partial sequence of the amino acid sequence of the protein antigen of the non-self pathogen.

By stating that the T cell response eliciting peptide "naturally comprises" a distinct amino acid residue is meant that the distinct amino acid residue is present at the same position within the corresponding naturally occurring T cell response eliciting peptide. Hence, a T cell response eliciting peptide that naturally comprises a cysteine residue means a peptide derived from a non-self pathogen, wherein the partial sequence of the amino acid sequence of a protein antigen of the non-self pathogen, which corresponds to the amino acid sequence of said T cell response eliciting peptide, comprises a cysteine residue at the same position.

When it is denoted that the T cell response eliciting peptide is provided "without amino acid sequence modification" with respect to the wild type pathogenic T cell epitope, it is meant that the amino acid sequence of the peptide is identical to the amino acid sequence of the corresponding naturally derivable T cell epitope of the non-self pathogen (i.e. the T cell epitope producible by cleavage of the corresponding naturally occurring non-self pathogen). In particular, the amino acid sequence of the peptide does not include additions, deletions or substitutions of amino acid residues when compared to the naturally occurring peptide.

The term "amino acid" as used herein denotes an organic molecule possessing an amino moiety located at α-position to a carboxylic group. Examples of amino acids include: arginine, glycine, ornithine, lysine, histidine, glutamic acid, asparagic acid, isoleucine, leucine, alanine, phenylalanine, tyrosine, tryptophane, methionine, serine, proline. The amino acid employed is optionally in each case the L-form. Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

**Table - Amino acids with specific properties**

| **Amino Acid** | **3-Letter** | **1- Letter** | **Side-chain polarity** | **Side-chain charge (pH 7.4)** |
|---|---|---|---|---|
| Alanine | Ala | A | Nonpolar | neutral |
| Arginine | Arg | R | basic polar | positive |
| Asparagine | Asn | N | Polar | neutral |
| Aspartic acid | Asp | D | acidic polar | negative |
| Cysteine | Cys | C | Nonpolar | neutral |
| Glutamic acid | Glu | E | acidic polar | negative |
| Glutamine | Gln | Q | Polar | neutral |
| Glycine | Gly | G | Nonpolar | neutral |
| Histidine | His | H | Basic polar | positive(10%) |
| | | | | neutral(90%) |
| Isoleucine | Ile | I | Nonpolar | neutral |
| Leucine | Leu | L | Nonpolar | neutral |
| Lysine | Lys | K | basic polar | positive |
| Methionine | Met | M | Nonpolar | neutral |
| Phenylalanine | Phe | F | Nonpolar | neutral |
| Proline | Pro | P | Nonpolar | neutral |
| Serine | Ser | S | Polar | neutral |
| Threonine | Thr | T | Polar | neutral |
| Tryptophan | Trp | W | Nonpolar | neutral |
| Tyrosine | Tyr | Y | Polar | neutral |
| Valine | Val | V | Nonpolar | neutral |

An "epitope" is the part of a protein antigen that is recognized by the immune system, e.g. by antibodies, B cells or T cells. A "T cell epitope" is presented on the surface of a cell, where it is bound to MHC molecules. T cell epitopes presentable by MHC I can be bound by the T cell receptor of cytotoxic CD8 T lymphocytes (CD8 T cells or CTLs). T cell epitopes presentable by MHC I are typically peptides of 8 to 12 amino acids in length. Hence, a "T cell response eliciting peptide that is presentable via MHC class I" as referred to herein means a pathogen-derived peptide (in one embodiment a peptide consisting of 8 to 12 amino acid residues) capable of binding to the MHC I complex (but not to MHC II) without requiring to be processed (e.g. by cleavage of one or more amino acid residues) in the target cell.

A "cleavable bond" within the terms of the invention relates to a bond within the immunoconjugate arranged between the target cell binding moiety and the T cell response eliciting peptide that allows the T cell response eliciting peptide to be split off in order to be directly presented by an MHC class I molecule. The cleavable bond is located in direct connection to the T cell response eliciting peptide. Cleavage of the T cell response eliciting peptide from the immunoconjugate results in release of the T cell response eliciting peptide of its original amino acid sequence and without any additional amino acid residues.

Dependent on the type of the cleavable bond, the T cell response eliciting peptide is released from the immunoconjugate upon cleavage either "traceless" or "non-traceless". By "non-traceless" release as used herein is meant that the released T cell response eliciting peptide includes traces of a linker (i.e. traces in the form a chemical moiety, apart from amino acid(s)), with the proviso that said traces do not interfere with MHC class I binding. It is expressly mentioned that within the terms of the present invention a "non-traceless release" as referred to herein does not include release of the T cell response eliciting peptide including additional amino acid residue(s). By "traceless" release as used herein is meant that the released T cell response eliciting peptide does not include any traces of a linker.

By "pH dependent cleavage" is meant that cleavage of the cleavable bond is triggered by a change of the pH. In one embodiment of the invention, pH dependent cleavage is triggered at a pH present within the endosomal compartment of the target cell. In one embodiment of the invention, pH dependent cleavage is triggered at pH 5.5 - 6.5.

By "enzymatic cleavage" as used herein the proteolytic cleavage of the immunoconjugate is meant, which in general occurs by hydrolysis of the peptide bond. Enzymatic cleavage is affected by proteases, e.g. furin. Creation of a cleavable bond within the immunoconjugate which is cleavable by enzymatic cleavage can be achieved for example by binding of the T cell response eliciting peptide to the target cell binding moiety via a peptide linker.

The binding of the T cell response eliciting peptide to the target cell binding moiety may be effected by a disulfide bond, an ester bond, a chemical linker, a peptide linker or by non-covalent binding (e.g. by biotin/streptavidin, biotin/theophylline).

By "covalent coupling" or "covalent binding" of the T cell response eliciting peptide to the target cell binding moiety is meant a coupling by a chemical bond involving the sharing of electron pairs between an atom of the T cell response eliciting peptide and an atom of the target cell binding moiety.

By "non-covalent coupling" or "non-covalent binding" of the T cell response eliciting peptide to the target cell binding moiety is meant a coupling that does not involve the sharing of electrons. Examples for non-covalent couplings include immunological bindings or bindings via biotin/avidin, biotin/streptavidin and the like. In one embodiment of an immunological binding is the coupling is affected via an antibody that is included in the immunoconjugate, wherein the antibody specifically binds to an organic molecule, which is bound to the T cell response eliciting peptide. To release the T cell response eliciting peptide after internalization, in this embodiment the T cell response eliciting peptide is coupled to said organic molecule via a cleavable bond. The organic molecule is preferably a small molecule with a molecular weight of up to 900 Da. Examples for such organic molecules include biotin and theophylline.

A "peptide linker" or "linker peptide" as used herein refers to a peptide with an amino acid sequence, which is preferably of synthetic origin. A peptide linker used herein either comprises a functional domain including a cleavage site, in one embodiment a protease cleavage site, or the peptide linker creates a cleavage site, in one embodiment a protease cleavage site, when fused to the T cell response eliciting peptide. The amino acid sequence of a peptide linker used herein typically has a length of 8 to 50 amino acid residues. In one embodiment the peptide linker used herein comprises a furin cleavage site and the amino acid sequence of the peptide linker comprises:
- R-X-R-R, with X being any amino acid residue, or
- R-X-K-R, with X being any amino acid residue.

A "translocation domain" within the terms of the invention is a structural protein domain, which is responsible for membrane translocation enabling access to the cytosol, or a functional part of said protein domain. An example for a translocation domain is domain II from Pseudomonas aeruginosa exotoxin A.

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. To administer an immunoconjugate according to the invention by certain routes of administration, it may be necessary to coat the immunoconjugate with, or co-administer the immunoconjugate with, a material to prevent its inactivation. For example, the immunoconjugate may be administered to a subject in an appropriate carrier, for example, liposomes, or a diluent. Pharmaceutically acceptable diluents include saline and aqueous buffer solutions.

A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

Regardless of the route of administration selected, the immunoconjugates of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, the carrier preferably is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "cancer" as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

The term "half maximal inhibitory concentration" ("IC50") denotes the concentration of a particular compound or molecule required for obtaining 50% inhibition of a biological process *in vitro.* IC50 values can be converted logarithmically to pIC50 values (-log IC50), in which higher values indicate exponentially greater potency. The IC50 value is not an absolute value but depends on experimental conditions e.g. concentrations employed. The IC50 value can be converted to an absolute inhibition constant (Ki) using the Cheng-Prusoff equation (Biochem. Pharmacol. (1973) 22:3099).

A "recombinant protein" is a protein which has been produced by a recombinantly engineered host cell. It is optionally isolated or purified.

In case the target cell binding moiety is a protein, the target cell binding protein is preferably produced by recombinant means. Methods for recombinant production of proteins are widely known in the state of the art and comprise protein expression in prokaryotic and eukaryotic cells with subsequent isolation of the protein and usually purification to a pharmaceutically acceptable purity. For the expression of the proteins in a host cell, nucleic acids encoding the protein or parts thereof are inserted into expression vectors by standard methods. Expression is performed in appropriate prokaryotic or eukaryotic host cells, like CHO cells, NS0 cells, SP2/0 cells, HEK293 cells, COS cells, PER.C6 cells, yeast, or E. coli cells, and the protein is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

### 2. Detailed description of the embodiments of the invention

Conjugates comprising a T cell specific pathogenic peptide and a protein specifically binding to the surface receptor on a target cell are known from EP 0 659 437 A1. However the efficiency of the MHC class I peptide presentation on the cell surface and T cell activation was low in the approach disclosed in EP 0 659 437 A1. The inventors of the present invention observed that, without being bound to this theory, this effect occurs due to the fact that the peptide containing conjugate disclosed in EP 0 659 437 A1 requires further processing within the target cell. This is limited by the problem that in most cases the exogenously provided antigen is not routed to the cytoplasm where it can be processed by proteosomes and transported further to the MHC class I pathway and presented on the cell surface. Another, supposedly additionally occurring, effect could be that the antigen processing machinery and presentation process especially within tumor cells is defective (Ferrone S., Advances in Cancer Research, 93 (2005) 189-234).

The present invention provides an approach to improve MHC class I presentation of T cell specific pathogenic peptides. According to the invention, the T cell response eliciting peptide released from the immunoconjugate in the endosomal compartment after internalization of the immunoconjugate is directly suitable for presentation via binding to the MHC class I complex (without requiring further processing or trimming within the target cell). The T cell response eliciting peptide is bound to MHC class I molecules in the endosomal compartment, proceeds to the surface of the target cell in context with MHC class I by endosome recycling pathway (Donaldson J.G., Nat Rev Mol Cell Biol. Sep 2009; 10(9): 597-608) and does not require transportation to the antigen processing machinery in the cytosol and further processing within the cell. Hence, the T cell response eliciting peptide and a target cell binding moiety are coupled with each other via a cleavable bond so as to release (after cleavage) the T cell response eliciting peptide consisting of its original amino acid sequence. In other words, when the T cell response eliciting peptide is cleaved from the immunoconjugate it does not comprise additional any amino acid residues or lack any amino acid residues when compared to the amino acid sequence of the naturally occurring T cell response eliciting peptide (i.e. the released T cell response eliciting peptide is of the same length as the corresponding naturally occurring T cell response eliciting peptide). Due to this, immunoconjugates according to the invention are better suited candidates to mediate CD8 T cell activation and induce cell death in target cells. With immunoconjugates according to the invention, target cell lysis is induced (a) with considerably reduced amounts of immunoconjugate required and (b) faster, specifically when compared to constructs from which a peptide is split off that requires further processing within the target cell.

The invention relates to an immunoconjugate comprising at least one T cell response eliciting peptide that is presentable via MHC class I coupled to a target cell binding moiety via a cleavable bond, wherein the cleavable bond is arranged such that upon cleavage said T cell response eliciting peptide, which is directly presentable via a MHC class I molecule, is released from the immunoconjugate, characterized in that the naturally occurring T cell response eliciting peptide comprises at least one cysteine residue and in that the unmodified T cell response eliciting peptide is covalently coupled to the target cell binding antibody via a disulfide bond. In one embodiment of the invention, the T cell response eliciting peptide is released in the endosomal compartment of the target cell. In another embodiment of the invention, the released T cell response eliciting peptide is presentable via MHC class I without further processing within the target cell.

In one embodiment of the invention the amino acid sequence of the released T cell response eliciting peptide is identical to an amino acid sequence of a naturally occurring T cell response eliciting peptide.

In one embodiment of the invention, the amino acid sequence of the released T cell response eliciting peptide is identical to the amino acid sequence of the T cell response eliciting peptide coupled to the target cell binding moiety.

In one embodiment of the invention the released T cell response eliciting peptide consists of the identical number of amino acids as the T cell response eliciting peptide coupled to the target cell binding protein and wherein the released T cell response eliciting peptide is suitable for presentation via the MHC class I complex.

In one embodiment of the invention the immunoconjugate comprises at least one T cell response eliciting peptide consisting of 8 - 12 amino acid residues that is presentable via MHC class I coupled to a target cell binding moiety via a cleavable bond, wherein the cleavable bond is arranged such that upon cleavage said T cell response eliciting peptide is released from the immunoconjugate, wherein the released T cell response eliciting peptide consists of the identical number of amino acids as the T cell response eliciting peptide bound to the target cell binding moiety.

In one embodiment of the invention, the T cell response eliciting peptide consists of 8 to 12 amino acids.

In one embodiment of the invention, the T cell response eliciting peptide is a naturally occurring T cell response eliciting peptide.

In one embodiment of the invention, the T cell response eliciting peptide comprises at least one cysteine residue. In one embodiment of the invention, the T cell response eliciting peptide is a naturally occurring T cell response eliciting peptide comprising at least one cysteine residue. In one embodiment of the invention, the T cell response eliciting peptide comprises an N- or C-terminal cysteine residue. In one embodiment of the invention, the T cell response eliciting peptide is a naturally occurring T cell response eliciting peptide comprising an Nor C-terminal cysteine residue.

In one embodiment of the invention the T cell response eliciting peptide comprises a cysteine residue at the N-terminal position (position 1 from N-terminal to C-terminal direction) of its amino acid sequence. In one embodiment of the invention the T cell response eliciting peptide naturally comprises a cysteine residue at the N-terminal position (position 1 from N-terminal to C-terminal direction) of its amino acid sequence.

In one embodiment of the invention, the T cell response eliciting peptide comprises a leucine residue at position 2 from N-terminal to C-terminal direction of its amino acid sequence. In one embodiment of the invention the T cell response eliciting peptide naturally comprises a leucine residue at position 2 from N-terminal to C-terminal direction of its amino acid sequence. In one embodiment of the invention the T cell response eliciting peptide consists of 8 - 12 amino acid residues and naturally comprises a leucine residue at position 2 from N-terminal to C-terminal direction of its amino acid sequence.

In one embodiment of the invention, the immunoconjugate includes up to five T cell response eliciting peptides. In one embodiment of the invention, the immunoconjugate includes up to ten T cell response eliciting peptides.

In one embodiment of the invention the T cell response eliciting peptide is derived from a non-self pathogen, which is recognized by immune cells of the host organism of the target cells. In one embodiment of the invention the T cell response eliciting peptide is derived from an intracellular or extracellular pathogen. In one embodiment of the invention the T cell response eliciting peptide is derived from an intracellular pathogen. In one embodiment of the invention the T cell response eliciting peptide is derived from a viral intracellular pathogen.

In one embodiment the T cell response eliciting peptide is selected from SEQ ID NO: 2 to SEQ ID NO: 5.

In one embodiment the T cell response eliciting peptide is derived from Epstein-Barr virus. In one embodiment the T cell response eliciting peptide is derived from Epstein-Barr virus and is selected from SEQ ID NO: 2 and SEQ ID NO: 4.

In one embodiment the T cell response eliciting peptide is derived from Human herpesvirus 5. In one embodiment the T cell response eliciting peptide is derived from Human herpesvirus 5 and is of SEQ ID NO: 5.

In one embodiment the T cell response eliciting peptide is derived from Influenza A. In one embodiment the T cell response eliciting peptide is derived from Influenza A and is of SEQ ID NO: 3.

In one embodiment the amino acid sequence of the T cell response eliciting peptide coupled to the target cell binding moiety is identical to a, preferably 8 to 12 amino acids long, partial sequence of the amino acid sequence of a protein antigen of a non-self pathogen (in one embodiment an intracellular pathogen).

The T cell response eliciting peptide is bound to the target cell binding moiety via a cleavable bond. In one embodiment of the invention, the cleavable bond is cleavable by chemical, pH dependent or enzymatic cleavage.

In one embodiment of the invention, the cleavable bond is cleavable after internalization of the immunoconjugate into the target cell.

In one embodiment of the invention, the cleavable bond is cleavable within the endosomal compartment of the target cell. In one embodiment of the invention, the cleavable bond is cleavable after internalization of the immunoconjugate into the target cell within the endosomal compartment of the target cell.

In one embodiment of the invention, the cleavable bond allows traceless release of the T cell response eliciting peptide from the immunoconjugate.

In one embodiment of the invention, the T cell response eliciting peptide is covalently coupled to the target cell binding moiety. In one embodiment of the invention, the cleavable bond is a disulfide bond. In one embodiment of the invention, the T cell response eliciting peptide comprises at least one cysteine residue and the cleavable bond is a disulfide bond. In one preferred embodiment of the invention the target cell binding moiety is a protein and a disulfide bond is formed between a cysteine residue of the T cell response eliciting peptide and a cysteine residue of the target cell binding protein. In another preferred embodiment, a peptide linker is present between the target cell binding moiety and the T cell response eliciting peptide. In this case, the disulfide bond is formed between a cysteine residue of the T cell response eliciting peptide and a cysteine residue of the peptide linker.

In one embodiment of the invention, the T cell response eliciting peptide and the target cell binding moiety are coupled via a cleavable linker moiety.

The immunoconjugate according to the invention comprises a target cell binding moiety in order to route the T cell response eliciting peptide to the target cell.

In one embodiment of the invention, the target cell binding moiety is capable of being internalized into the target cell.

In one embodiment of the invention, the target cell binding moiety binds to a cell surface antigen of the target cell.

In one embodiment of the invention, the target cell is a tumor cell. In another embodiment of the invention, wherein the target cell is a tumor cell, the target cell binding moiety is capable of specifically binding to a surface-expressed tumor specific antigen (TSA, which is present only on tumor cells and not on other cells) or a surface-expressed tumor associated antigen (TAA, which are present on tumor cells and to a lesser extent on some other cells).

In one embodiment of the invention the target cell is a cell associated with disease severity in an infectious disease, an autoimmune disease, diabetes or allergy.

In one preferred embodiment of the invention the target cell binding moiety is an antibody.

In one embodiment, the target cell binding moiety specifically binds to CD19 or CDCP-1. In one embodiment, the target cell binding moiety specifically binds to CD19. In one embodiment, the target cell binding moiety specifically binds to CDCP-1.

In one preferred embodiment of the invention the target cell binding moiety is an antibody specifically binding to CD19 or CDCP-1. In one preferred embodiment of the invention the target cell binding moiety is an antibody specifically binding to CD19. In one preferred embodiment of the invention the target cell binding moiety is an antibody specifically binding to CDCP-1.

In one embodiment of the invention, the immunoconjugate is capable of being internalized into the target cell.

According to the invention, the T cell response eliciting peptide is directly presentable by MHC class I and does not require further processing within the target cell. Therefore, in one embodiment of the invention the immunoconjugate does not comprise a translocation domain.

With the invention, an immunoconjugate is provided which allows release of a T cell response eliciting peptide which is directly presentable via MHC class I. As the released T cell response eliciting peptide does not require delivery to the antigen processing machinery in the cytosol, the T cell response eliciting peptide are presented on the surface of the target cell in a short amount of time. Hence, in one embodiment of the invention, in less than 12 hours after internalization of the immunoconjugate into the target cell the T cell response eliciting peptide is presented on the surface of the target cell via an MHC class I molecule. In another embodiment of the invention, in less than 6 hours after internalization of the immunoconjugate into the target cell the T cell response eliciting peptide is presented on the surface of the target cell via an MHC class I molecule. In another embodiment of the invention, the immunoconjugate is capable of mediating T cell cytotoxicity against the target cell within less than 6 hours after internalization of the immunoconjugate into the target cell.

The invention further relates to a pharmaceutical composition comprising an immunoconjugate according to the invention. One embodiment of the invention is a pharmaceutical composition comprising an immunoconjugate according to the invention in combination with a pharmaceutically acceptable carrier.

Another aspect of the invention is the use of a T cell response eliciting peptide that is presentable via MHC class I as further described and defined above for the generation of an immunoconjugate according to the invention.

Disclosed herein is the use of an immunoconjugate according to the invention for specifically inducing T cell cytotoxicity against the target cell. In one embodiment of this disclosure the immunoconjugate is used for specifically inducing cell-mediated immunity via CD8 T cells against a target cell. In one embodiment of this disclosure the immunoconjugate is internalized into the target cell and the T cell response eliciting peptide is released from the immunoconjugate within the endosomal compartment of the target cell. In one embodiment of this disclosure in less than 12 hours after internalization of the immunoconjugate into the target cell the T cell response eliciting peptide is presented on the surface of the target cell via an MHC class I molecule. In on eembodiment of this disclosure in less than 6 hours after internalization of the immunoconjugate into the target cell the T cell response eliciting peptide is presented on the surface of the target cell via an MHC class I molecule.

In another embodiment of this disclosure, the immunoconjugate is used for of mediating T cell cytotoxicity against the target cell within less than 6 hours after internalization of the immunoconjugate into the target cell.

Another aspect of the invention is a method for the generation of an immunoconjugate according to the invention, comprising the steps of
a) providing the recombinant target cell binding moiety,
b) providing the T cell response eliciting peptide, and
c) coupling of at least one of said T cell response eliciting peptide to said target cell binding moiety via the cleavable bond.

One embodiment of the invention relates to a method for the generation of an immunoconjugate, wherein an unmodified T cell response eliciting peptide is coupled to the target cell binding moiety. In other words, one embodiment of the invention relates to a method for the generation of an immunoconjugate wherein the T cell response eliciting peptide is provided without amino acid sequence modifications and is coupled to the target cell binding moiety.

Also disclosed herein is a method for the production of an immunoconjugate according to the invention for specifically inducing cell-mediated immunity via CD8 T cells against a target cell, comprising the steps of
a) providing the recombinant target cell binding moiety,
b) providing the T cell response eliciting peptide, and
c) coupling of at least one of said T cell response eliciting peptide to said target cell binding moiety via the cleavable bond. In one embodiment of this disclosure an unmodified T cell response eliciting peptide is coupled to the target cell binding moiety.

Also disclosed herein is the use of an immunoconjugate according to the invention for the manufacture of a pharmaceutical composition. Also disclosed herein is a method for the manufacture of a pharmaceutical composition comprising an immunoconjugate according to the invention, including formulating the immunoconjugate according to the invention in combination with at least one pharmaceutically acceptable carrier.

Another aspect of the invention is the immunoconjugate according to the invention for use as a medicament. Another aspect of the invention is the immunoconjugate according to the invention comprising a cancer cell binding moiety, for the treatment of cancer.

Another aspect of the invention is the immunoconjugate according to the invention for use in the treatment of infectious diseases, autoimmune diseases, diabetes or allergy.

Another aspect of the invention is the immunoconjugate according to the invention for use in specifically inducing cell-mediated immunity via CD8 T cells against the target cells.

Also disclosed herein is the pharmaceutical composition according to the invention for use as a medicament. Also disclosed herein is the pharmaceutical composition according to the invention, wherein the immunoconjugate comprised in the pharmaceutical composition comprises a cancer cell binding moiety, for the treatment of cancer. Also disclosed herein is the pharmaceutical composition according to the invention for use in the treatment of infectious diseases, autoimmune diseases, diabetes or allergy. Also disclosed herein is the pharmaceutical composition according to the invention for use in specifically inducing cell-mediated immunity via CD8 T cells against the target cells.

Also disclosed herein is the use of an immunoconjugate according to the invention for the manufacture of medicament. Also disclosed herein is the use of an immunoconjugate according to the invention for the manufacture of medicament for the treatment of cancer.

Also disclosed herein is the use of an immunoconjugate according to the invention for the manufacture of medicament for the treatment of infectious diseases, autoimmune diseases, diabetes or allergy. Also disclosed herein is the use of an immunoconjugate according to the invention for the manufacture of medicament for specific induction of cell-mediated immunity via CD8 T cells against the target cells.

Also disclosed herein is a method of treatment of a patient suffering from a disease by administering an immunoconjugate according to the invention to the patient in the need of such treatment. Also disclosed herein is a method of treatment of a patient suffering from cancer by administering an immunoconjugate according to the invention comprising a cancer cell binding moiety to the patient in the need of such treatment. Also disclosed herein is a method of treatment of a patient suffering from an infectious disease, an autoimmune disease, diabetes or allergy by administering an immunoconjugate according to the invention to the patient in the need of such treatment.

Also disclosed herein is a method of treatment of a patient suffering from a disease by administering a pharmaceutical composition according to the invention to the patient in the need of such treatment. Also disclosed herein is a method of treatment of a patient suffering from cancer by administering a pharmaceutical composition according to the invention to the patient in the need of such treatment Also disclosed herein is a method of treatment of a patient suffering from an infectious disease, an autoimmune disease, diabetes or allergy by administering a pharmaceutical composition according to the invention to the patient in the need of such treatment..

### 3. Specific embodiments of the invention

In the following specific embodiments of the invention are listed.
1. An immunoconjugate comprising at least one naturally occurring T cell response eliciting peptide that is presentable via MHC class I coupled to a target cell binding antibody via a cleavable bond, wherein the cleavable bond is arranged such that upon cleavage said T cell response eliciting peptide, which is directly presentable via a MHC class I molecule, is released from the immunoconjugate, characterized in that the naturally occurring T cell response eliciting peptide comprises at least one cysteine residue and in that the unmodified T cell response eliciting peptide is covalently coupled to the target cell binding moiety via a disulfide bond.
2. The immunoconjugate according to embodiment 1, wherein the amino acid sequence of the released T cell response eliciting peptide is identical to an amino acid sequence of a naturally occurring T cell response eliciting peptide.
3. The immunoconjugate according to any one of the preceding embodiments, wherein the T cell response eliciting peptide comprises an N- or C-terminal cysteine residue.
4. The immunoconjugate according to any one of the preceding embodiments, wherein the T cell response eliciting peptide comprises a leucine residue at position 2 (counted from N-terminal to C-terminal direction).
5. The immunoconjugate according to any one of the preceding embodiments, comprising up to five T cell response eliciting peptides.
6. The immunoconjugate according to any one of the preceding embodiments, wherein the T cell response eliciting peptide consists of 8 to 12 amino acids.
7. The immunoconjugate according to any one of the preceding embodiments, wherein the T cell response eliciting peptide is derived from Epstein-Barr virus, Human herpesvirus 5 or Influenza A.
8. The immunoconjugate according to any one of the preceding embodiments, wherein the T cell response eliciting peptide is derived from Epstein-Barr virus.
9. The immunoconjugate according to any one of the preceding embodiments, wherein the T cell response eliciting peptide is derived from Human herpesvirus 5.
10. The immunoconjugate according to any one of the preceding embodiments, wherein the T cell response eliciting peptide is derived from Influenza A.
11. The immunoconjugate according to any one of the preceding embodiments, wherein the T cell response eliciting peptide is selected from SEQ ID NO: 2 to SEQ ID NO: 5.
12. The immunoconjugate according to any one of the preceding embodiments, wherein the cleavable bond is cleavable after internalization of the immunoconjugate into the target cell.
13. The immunoconjugate according to any one of the preceding embodiments, wherein the cleavable bond is cleavable within the endosomal compartment of the target cell.
14. The immunoconjugate according to any one of the preceding embodiments, wherein the target cell binding moiety is capable of being internalized into the target cell.
15. The immunoconjugate according to any one of the preceding embodiments, wherein the target cell binding moiety binds to a cell surface antigen of the target cell.
16. The immunoconjugate according to any one of the preceding embodiments, wherein the target cell is a tumor cell.
17. The immunoconjugate according to any one of the preceding embodiments, wherein the target cell binding antibody specifically binds to CD19 or CDCP-1.
18. The immunoconjugate according to any one of the preceding embodiments, wherein the target cell binding antibody specifically binds to CD19.
19. The immunoconjugate according to any one of the preceding embodiments, wherein the target cell binding antibody specifically binds to CDCP-1.
20. The immunoconjugate according to any one of the preceding embodiments capable of being internalized into the target cell.
21. The immunoconjugate according to any one of the preceding embodiments, wherein the immunoconjugate does not comprise a translocation domain.
22. The immunoconjugate according to any one of the preceding embodiments, wherein in less than 12 hours after internalization of the immunoconjugate into the target cell the T cell response eliciting peptide is presented on the surface of the target cell via an MHC class I molecule.
23. A pharmaceutical composition comprising an immunoconjugate according to any one of the preceding embodiments in combination with a pharmaceutically acceptable carrier.
24. Use of a T cell response eliciting peptide that is presentable via MHC class I for the generation of an immunoconjugate according to any one of embodiments 1 to 22.
25. A method for the generation of an immunoconjugate according to any one of embodiments 1 to 22, comprising the steps of
   ∘ providing the recombinant target cell binding moiety,
   ∘ providing the T cell response eliciting peptide,
   ∘ coupling of at least one of said T cell response eliciting peptide to said target cell binding moiety via the cleavable bond.
26. The method according to embodiment 25, wherein an unmodified T cell response eliciting peptide is coupled to the target cell binding moiety.
27. The immunoconjugate according to one of embodiments 1 to 22 for use as a medicament.
28. The immunoconjugate according to one of embodiments 1 to 22 comprising a cancer cell binding moiety, for the treatment of cancer.
29. The immunoconjugate according to one of embodiments 1 to 22 for the treatment of infectious diseases, autoimmune diseases, diabetes or allergy.

### DESCRIPTION OF THE AMINO ACID SEQUENCES

| | |
|---|---|
| **SEQ ID NO:1** | Human herpesvirus 5 (CMV) peptide pp65 495-503 |
| | NLVPMVATV |
| **SEQ ID NO:2** | Epstein-Barr virus (EBV) peptide LMP-2 426-434 |
| | CLGGLLTMV |
| **SEQ ID NO:3** | Influenza A (FLU) peptide NP 44-52 |
| | CTELKLSDY |
| **SEQ ID NO:4** | Epstein-Barr virus (EBV) peptide BLMF-1 280-288 |
| | GLCTLVAML |
| **SEQ ID NO:5** | Human herpesvirus 5 (CMV) peptide 309-317 |
| | CRVLCCYVL |
| **SEQ ID NO:6** | modified Human herpesvirus 5 (CMV) peptide pp65 495-503 (cCMV peptide) |
| | CNLVPMVATV |
| **SEQ ID NO:7** | modified Human herpesvirus 5 (CMV) peptide pp65 495-503 (CMVc peptide) |
| | NLVPMVATVC |
| **SEQ ID NO:8** | modified Human herpesvirus 5 (CMV) peptide pp65 495-503 (rcCMV peptide) |
| | CLVPMVATV |
| **SEQ ID NO:9** | modified Human herpesvirus 5 (CMV) peptide pp65 495-503 (CMVrc peptide) |
| | NLVPMVATC |
| **SEQ ID NO:10** | modified Human herpesvirus 5 (CMV) peptide pp65 495-503 (CMVpen peptide) |
| | NLVPMVATX, wherein X = penicillamine, a modified valine residue, wherein a SH-group is bound to Cβ |

### EXAMPLES

The following examples are provided to aid the understanding of the present invention, the true scope of which is set forth in the appended claims.

### Example 1:

### Influence of C- or N-terminal modification of CMV-peptide on binding to MHC class I (HLA-A2) and CMV-specific T cell activation

In order to assess the impact of C- or N-terminal amino acid modifications included in a naturally occurring T cell response eliciting peptide, naturally processed CMV peptide of the amino acid sequence NLVPMVATV (SEQ ID NO. 1) was subjected to the following modifications:

| | |
|---|---|
| cCMV | addition of a cysteine residue at the N-terminus of the peptide |
| | (peptide according to SEQ ID NO: 6) |
| CMVc | addition of a cysteine residue at the C-terminus of the peptide |
| | (peptide according to SEQ ID NO: 7) |
| rcCMV | substitution of the N-terminal amino acid of the peptide by a cysteine residue |
| | (peptide according to SEQ ID NO: 8) |
| CMVrc | substitution of the C-terminal amino acid of the peptide by a cysteine residue |
| | (peptide according to SEQ ID NO: 9) |
| CMVpen | substitution of the C-terminal valine residue by penicillamine |
| | (peptide according to SEQ ID NO: 10) |
| | 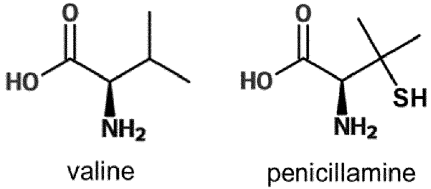 |

The peptides were synthesized using standard Fmoc-chemistry. Amino acids used were all L-isomers.

### Example 1A

### Generation of CMV peptide specific T cells from peripheral blood of healthy donors

The frequency of CMV peptide specific CD8 T cells in fresh isolated PBMCs from healthy donors or in T cell cultures was investigated in FACS via pentamer staining.

Briefly, aliquots of 3-5x10E05 T cell cultures or freshly isolated PBMCs from HLA-A2-matched healthy donors were washed with BD FACS buffer and incubated with 100 µL BD FACS buffer comprising 5 µL human TruStain FcX (BioLegend, 422302) for 15 min at room temperature. Afterwards 90 µL BD FACS buffer comprising 10µL of biotin conjugated CMV-Pentamer (ProImmune, F008-4, 1:10) was added and incubated for 1h at 4°C.

Upon washing with BD FACS buffer cells were stained in 100 µl BD FACS buffer with FITC-labelled anti-CD8a mAb (clone LT8, ProImmune) as well as streptavidin-APC conjugate at 4°C for 20 min. After 3x washing with BD FACS buffer and centrifugation cells were resuspended in 200 µl BD FACS buffer containing 1 µg/ml DAPI and MFI signals of stained cells were analyzed by BD Biosciences FACSCanto II flow cytometer. Cells from HLA-A1 donors were used for assessment of unspecific binding of tetramers.

Freshly isolated PBMC were used for stimulation on the day of isolation with CMV derived peptide NLVPMVATV (SEQ ID NO: 1) as follows:
2x10E5 cells per 96-well in 200µL RPMI+ medium containing 1µM peptide were incubated for three days in 96 well plates. On day 3 the plates were centrifuged at 300g for 2 min. and 160µl supernatant were removed from wells and replased with fresh RPMI+ medium containing 1µM peptide and 100U/mL IL-2. At day 8 and 10 the medium was replaced again as described at day 3. The cultured cells were splitted at day 14 without stimulation with peptide. At day 16 the cell culture was restimulatated with autologous PBMCs (irradiated with 11 Gray) and pulsed with CMV peptide as follows: 12x10E6 T-cells per well in 2000µL RPMI+ medium containing 1µM peptide plus 8,7x10E6 irradiated PBMCs per well in 6 well plate. At days 19, 21 and 25 the culture was splitted again and stimulated as described above at day 30. At days 35, 37, 41 the culture was splitted and on day 44 restimulated according the protocol. The culture was tested for the frequency of the CMV peptide specific cells and used at day 51 in the LDH cytotoxicity assay and at day 54 in the ELISPOT assay for the specificity against the cells loaded with the indicated CMV peptide.

### Example 1B

### Evaluation of CMV peptide specific T cell activation after treatment of tumor cells with free CMV peptides by IFNγ-ELISPOT

HLA-A2 restricted CMV peptide specific T cell activation was evaluated by IFNγ-ELISPOT on different tumor cell lines expressing different levels of relevant MHC I molecules on the surface:
- MDA-MB 231: HLA-A2 high
- HCT-116: HLA-A2 low
- T2: a TAP deficient cell line, which does not express unloaded MHC class I molecules on the surface on the cells

In brief, 10,000 tumor cells were respectively treated with different concentrations of the free modified CMV peptides (cCMV, CMVc, rcCMV, CMVrc, CMVpen) or the naturally occurring, unmodified CMV peptide (in concentrations of 13,245 µM or 1,3245 µM) in presence of 500 CMV-specific CD8 T cells obtained in example 1A. As controls, tumor cells and T cells were incubated in absence of free peptide and, as a further control, T cells were incubated alone, under the same conditions.

The experiments were carried out either using serum-containing RPMI+ Medium or serum-free AIM-V medium.

IFNγ-ELISPOT was performed as follows:
On day 1, plates were washed with sterile PBS. Subsequently, 50µL RPMI+ medium was added per well and the plates were incubated for 20-30 min. Subsequently, peptides were added 50µL RPMI+ to the respective wells. The respective tumor cells were harvested using Accutase. 10.000 tumor cells per well were added in 100µL RPMI+ in the respective wells. After incubation for 20 min plates were incubated in the dark for about 6 hours. T cells were filtered through 40µm mash and washed once in RPMI+ (centrifuged at 300g, 2 min). 50 µL RPMI+ medium containing 500 peptide-specific CD8 T cells were added. Plates were incubated overnight.

On day 2, plates were emptied and washed with PBS. Anti-human IFN-γ monoclonal antibody (Mabtech 7-B6-ALP) was diluted 1:200 in filtered PBS / 0.5 % FCS. 100µL pf diluted antibody was added per well and incubated for 2 hours. Subsequently, the plates were washed with PBS. Substrate solution (BCIP/NBT-plus) was filtered through a 0.45µm filter. 100µL substrate solution was added per well and developed until distinct spots emerged. After stopping the development by washing in tap water the plates were dried in the dark.

The plates were analyzed in an ELISPOT Reader (Cellular Technology Ltd.) using the "ImmunoSpot 5.0 professional DC" software.

The composition of the RPMI+ medium was as follows:

| | |
|---|---|
| 500mL | RPMI Medium 1640 (Life Technologies) |
| 8% | Human serum (heat-inactivated and filtered) |
| 1% | L-Glutamine |
| 1% | NEAA |
| 1% | Sodium pyruvate |
| 0,2% | PenStrep |
| 0,1% | β-mercaptoethanol |

Results are indicated in Fig. 1.

It was observed that N-terminal modification of the CMV-peptide lead to a loss of T cell activation. C-terminal modification diminished the activation of T cells. In addition, the modification of a side chain of an amino acid naturally present within the CMV peptide's amino acid sequence lead to a loss of T cell activation. Hence, it can be concluded that modifications within a naturally occurring T cell response eliciting peptide may impact (i.e. diminish) loading on MHC class I molecules and, accordingly, T cell activation.

However, when using naturally occurring T cell response eliciting peptides, optimal MHC class I binding and loading can be assured.

### Example 2:

### Proposed mode of action of immunoconjugates according to the invention

Fig. 2 demonstrates a proposed mode of action of an immunoconjugate according to the invention. Exemplarily, an antibody is shown as target cell binding moiety, however other moieties are equally suitable.

In steady state, the target cell surface (e.g. the surface of a tumor cell) is decorated with the target antigen, to which the target cell binding moiety specifically binds. In addition, MHC class I molecules presenting self peptides are expressed on the cell surface.

Without being bound to this theory, binding of the immunoconjugate to the target antigen **(A)** leads to internalization of the target antigen-immunoconjugate complex **(B)** and subsequent release of the T cell response eliciting peptide from the immunoconjugate in the early endosomal compartment **(C)**. Importantly, the released peptide consists of an amino acid sequence identical to its corresponding naturally occurring peptide and does not comprise any amino acid modifications, specifically no C- or N- terminal amino acid modifications.

Self peptides present on MHC class I molecules are passively exchanged by the non-self peptide (i.e. the T cell response eliciting peptide cleaved off from the immunoconjugate) within the endosomal compartment **(D)**. The peptide-loaded MHC class I molecules are routed to the cell surface via endosomal trafficking **(E)** leading to presentation of the T cell response eliciting peptide on the target cell surface in context of MHC class I.

Peptide-specific CD8 T cells recognize and bind to the peptide-loaded MHC class I molecules on the target cell (F) leading to specific induction of T cell cytotoxicity against the target cell.

### Example 3:

### Conjugation of a monoclonal anti-CDCPl antibody with at least one T cell response eliciting peptide that is presentable via MHC class I

To generate an immunoconjugate according to the invention, a monoclonal antibody (mAb) specifically binding to CUB domain-containing protein 1 (CDCP1, also designated as CD318, disclosed in EP 1 396 501 A1) was coupled to either one of the following pathogen derived peptides:
- Epstein-Barr virus (EBV) peptide 426-434 (SEQ ID NO: 2, CLGGLLTMV), or
- Influenza A NP (FLU) peptide 44-52 (SEQ ID NO: 3, CTELKLSDY).

The peptides were synthesized using standard Fmoc-chemistry. Amino acids used were all L-isomers.

### Example 3A:

### Production of immunoconjugates comprising monoclonal anti-CDCPl antibody and EBV and/or FLU peptide, wherein the peptides are coupled to the antibody via a cleavable S-S bond

For production of examples of immunoconjugates according to the invention, wherein the immunoconjugate comprises a disulfide bond in order to release the peptide upon internalization into the target cell, the peptides were conjugated to the antibody as follows:
The αCDCP1-mAb was first derivatized in 0.1 M potassium phosphate buffer at pH 7.2 in presence of 10 eq. of N-Succinimidyl 3-(2-pyridyldithio)-propionate (SPDP, Pierce). After 2 hours reaction time the derivatized antibody was purified by gel filtration into 0.1 M potassium phosphate buffer containing 10 mM EDTA at pH 7.0.

In order to produce immunoconjugates comprising either EBV or FLU peptide, 10 eq. of the respective peptide was added to the derivatized antibody and reacted overnight. The immunoconjugates according to the invention were purified by gel filtration.

In order to produce immunoconjugates comprising EBV and FLU peptide, the derivatized antibody was reacted with 5 eq. of each, EBV and FLU peptide, overnight. The immunoconjugates according to the invention were purified by gel filtration.

Produced immunoconjugates according to the invention are herein referred to as follows:
- <CDCP1-SS-pEBV> immunoconjugate comprising anti-CDCPl MAb coupled to EBV peptide 426-434 by a disulfide bond
- <CDCP1-SS-pFLU> immunoconjugate comprising anti-CDCPl MAb coupled to FLU peptide 44-52 by a disulfide bond
- <CDCP1-SS-pEBV/FLU> immunoconjugate comprising anti-CDCPl MAb coupled to EBV peptide 426-434 and FLU peptide 44-52 by respective disulfide bonds

### Example 3B (comparison):

### Production of immunoconjugates comprising monoclonal anti-CDCPl antibody and EBV or FLU peptide, wherein the peptides are coupled to the antibody via a non-cleavable thioether bond

For comparative analyses the peptides were conjugated to the antibody in a non-cleavable manner via a thioether bond:
The αCDCP1-mAb was first derivatized in 0.1 M potassium phosphate buffer at pH 7.2 in presence of 10 eq. of N-epsilon-Malemidocaproyl-oxysuccinimide ester (EMCS, Pierce). After 2 hours reaction time the derivatized antibody was purified by gel filtration into 0.1 M potassium phosphate buffer containing 10 mM EDTA at pH 7.0.

Afterwards 10 eq. of the respective peptide was added to the derivatized antibody and reacted overnight. The immunoconjugates was purified by gel filtration.

Produced immunoconjugates used for comparison are herein referred to as follows:
- <CDCP1-S-pEBV> immunoconjugate comprising anti-CDCPl MAb coupled to EBV peptide 425-433 by a thioether bond
- <CDCP1-S-pFLU> immunoconjugate comprising anti-CDCPl MAb coupled to FLU peptide 44-52 by a thioether bond

### Example 3C:

### MS analytics and stability evaluation of immunoconjugates comprising monoclonal anti-CDCP1 antibody coupled to EBV and/or FLU peptide

The labeling rate of the immunoconjugates formed in example 3A and 3B were analyzed by LC-ESI mass spectrometry.

**Sample preparation:** The IgG or labelled-IgG was deglycosylated with N-glycosidase F before measuring. The IgG or labelled-IgG was alternatively reduced with TCEP after deglycosylation in order to measure the light chain and the heavy chain separately.

**Analytics** were performed using an LC-ESI-MS (Waters) HPLC System with a reverse-phase column with a water : acetonitrile gradient and ESI-TOF-MS measurement and detection. MS Data Analysis was performed using MassLynx Software (Waters).

The analyses showed that at average of 0.8 peptides were coupled to each antibody molecule (data not shown).

In order to check the stability of the immunoconjugates, stability within the stock solution and within cell culture medium was assessed. Stock solutions of immunoconjugates were filtrated by spin columns (MW cutoff 50 kDa) and analyzed by LC-MS. No free peptide was detected. Cell culture medium which was spiked with immunoconjugates was analyzed by LC-MS. No free peptide was detected. As a positive control, cell culture medium was spiked with free peptide.

### Example 3D:

### Cellular binding studies of immunoconjugates comprising monoclonal anti-CDCP1 antibody coupled to EBV and/or FLU peptide to CDCP1 expressing MDA-MB 231 cells

In order to evaluate binding of the immunoconjugates to their respective antigen, binding of the immunoconjugates of examples 3A and 3B to CDCP1 expressing MDA-MB 231 cells was assessed in a FACS based binding assay.

Briefly, MDA-MB 231 cell were detached via treatment with accutase, separated from the detaching media by centrifugation, and suspended in MDA-MB 231 cell medium at a concentration of 4 x 10E06 cells/ml. 50 µl cell aliquots were incubated with serial dilutions (20µg/ml - 0,02 µg/ml immunoconjugate in BD FACS buffer) of immunoconjugate or unconjugated anti-CDCP1 antibodies (parental anti-CDCPl antibodies) for 30 min at 4 °C. Following washing with BD FACS buffer cells were stained with FITC labeled anti-human IgG H+L (Invitrogen A11013; 10 µg/ml in BD FACS buffer) for 30 min at 4°C. After washing and centrifugation MFI signals of stained cells were analyzed by BD Biosciences FACSCanto flow cytometer (Figure 3).

The results from the FACS based cellular binding studies shown in Figure 3 demonstrate that all immunoconjugates bind to CDCP1 expressing MDA-MB 231 tumor cells in a comparable fashion.

### Example 3E:

### Binding studies of immunoconjugates comprising monoclonal anti-CDCP1 antibody coupled to EBV and/or FLU peptide to the extracellular domain (ECD) of CDCP1 by surface plasmon resonance

Biochemical binding of the immunoconjugates of examples 3A and 3B to human CDCP1-ECD was investigated by surface plasmon resonance using a BIACORE T100 instrument (GE Healthcare).

Around 2000 resonance units (RU) of the capturing system (10 µg/ml goat anti human IgG Fc Fragment specific; Jackson Immuno Research) were coupled on a CM4 chip (GE Healthcare, BR-1005-34) at pH 5.0 by using an amine coupling kit supplied by the GE Healthcare. Running buffer for immobilization was HBS-N pH 7.4 (10 mM HEPES, 150 mM NaCl, pH 7.4, GE Healthcare, BR-1006-70). For the followed kinetic assay running and dilution buffer was HBS-P pH 7.4 (10 mM HEPES, 150 mM NaCl, 0,05% Surfactant P20, pH 7.4, GE Healthcare, BR-1006-71).

The flow cell was set to 25 °C - and the sample block set to 12 °C - and primed with running buffer twice. The immunoconjugates and for comparison, the parental anti-CDCPl mAb antibody ((RO5464169-000-0004) were captured by injecting a 1 µg/ml solution for 60 sec at a flow of 10 µl/min.

Association was measured by injection of human CDCP1-ECD in various concentrations in solution for 180 sec at a flow of 30 µl/min starting with 1350 nM, followed by one 1:1.5 dilution and further in 1:3 dilutions. The dissociation phase was monitored for up to 300 sec and triggered by switching from the sample solution to running buffer. The surface was regenerated by washing with two consecutive injections of a Glycine pH 1.7 solution for 60 sec at a flow rate of 10 µl/min. Bulk refractive index differences were corrected by subtracting the response obtained from a goat anti human IgG Fc surface. Blank injections are also subtracted (= double referencing). For calculation of KD and other kinetic parameters the Langmuir 1:1 model was used.

The results are shown in Table 1:

**Table 1: Binding of immunoconjugates to CDCP1 as assessed by Biacore®**

| **immunoconjugate** | **k_{D} [s⁻¹]** |
|---|---|
| parental αCDCP1-mAb | 5.6E-03 |
| <CDCP1-SS-pEBV> | 4.6E-03 |
| <CDCP1-SS-pFLU> | 5.5E-03 |
| <CDCP1-SS-pEBV/FLU> | 5.2E-03 |
| <CDCP1-S-pEBV> | 5.0E-03 |
| <CDCP1-S-pFLU> | 5.1E-03 |

The results from Biacore studies shown in Table 1 demonstrate that all immunoconjugates bind to CDCP1-ECD in a comparable fashion as compared to parental anti-CDCPl reference antibody.

### Example 3F:

### Internalization studies of immunoconjugates comprising monoclonal anti-CDCP1 antibody coupled to EBV and/or FLU peptide into CDCP1 expressing human tumor cells (MDA-MB 231)

For the evaluation of internalizing capacity of generated immunoconjugates in comparison to parental anti-CDCPl mAb a FACS based internalization assay with the CDCP1 expressing human tumor cell line MDA-MB 231 was performed.

Briefly, MDA-MB 231 cell were detached via treatment with accutase, separated from the detaching media by centrifugation, and suspended in MDA-MB 231 cell cuture medium (RPMI / 2mM Glutamin / 10% FCS) at a concentration of 3 x 10E06 cells/ml. 50 µl cell aliquots were incubated with immunoconjugates or parental antibodies (5µg/ml in MDA-MB 231 cell culture medium) for 30 min at 4°C. Following 2x washing with cold MDA-MB 231 cell culture medium (RPMI / 2mM Glutamin / 10% FCS) cells were incubated in 100 µl medium at 4°C or 37°C for 30, 60, 120, 240 min and overnight [23h].

After the mentioned time points cells were washed, centrifuged and stained with Alexa488 labeled anti-human IgG H+L (Invitrogen A11013; 10µg/ml in BD FACS buffer) for 30 min at 4°C. After 2x washing and centrifugation cells were held in 100 µl BD Cell-Fix buffer and MFI signals of stained cells were analyzed by BD Biosciences FACSCanto flow cytometer.

No internalization was detected upon incubation at 4 °C. The results for the internalization studies at 37 °C are shown in Table 2.

**Table 2: Internalization of immunoconjugates comprising monoclonal anti-CDCP1 antibody coupled to EBV and/or FLU peptide into CDCP1 expressing human tumor cells (MDA-MB 231) at 37 °C**

| | **[%] Internalization at 37 °C after** | | | | |
|---|---|---|---|---|---|
| | **30 min** | **60 min** | **120 min** | **240 min** | **23 h** |
| anti-CDCP1 MAb (positive control) | 30 | 39 | 55 | 68 | 87 |
| <CDCP1-SS-pEBV> | 27 | 35 | 50 | 63 | 87 |
| <CDCP1-SS-pEBV/FLU> | 27 | 35 | 54 | 65 | 87 |
| <CDCP1-S-pEBV> | 26 | 36 | 52 | 66 | 88 |
| anti-human IgG (negative control) | 0 | 0 | 0 | 0 | 0 |

Results from the FACS based internalization studies show that all tested immunoconjugates internalize into CDCP1 expressing MDA-MB 231 tumor cells comparable to parental anti-CDCPl reference antibody (RO5464169-000-0004).

### Example 4:

### Conjugation of a monoclonal anti-CD19 antibody with at least one T cell response eliciting peptide that is presentable via MHC class I

### Example 4A:

### Production of immunoconjugates comprising monoclonal anti-CD19 antibody and EBV and/or FLU peptide, wherein the peptides are coupled to the antibody via a cleavable S-S bond

A mAb specifically binding to CD19 (in house preparation purified from hybridoma supernatant) was coupled to either one of the peptides used in example 3A.

For production of examples of immunoconjugates according to the invention, wherein the immunoconjugate comprises a disulfide bond in order to release the peptide upon internalization into the target cell, the peptides were conjugated to the antibody via a disulfide bond as described in example 3A.

Produced immunoconjugates according to the invention are herein referred to as follows:
- <CD19-SS-pEBV> immunoconjugate comprising anti-CD19 MAb coupled to EBV peptide 426-434 by a disulfide bond
- <CD19-SS-pFLU> immunoconjugate comprising anti-CD19 MAb coupled to FLU peptide 44-52 by a disulfide bond
- <CD19-SS-pEBV/FLU> immunoconjugate comprising anti-CD 19 MAb coupled to EBV peptide 426-434 and FLU peptide 44-52 by a disulfide bond

### Example 4B:

### Cellular binding studies of immunoconjugates comprising monoclonal anti-CD19 antibody coupled to EBV and/or FLU peptide to CD19 expressing RL non-Hodgkin's lymphoma cells

In order to evaluate binding of the immunoconjugates to their respective antigen, binding of the immunoconjugates of example 4A to CD19 expressing RL cells was assessed in a FACS based binding assay.

Briefly, RL cell were cultivated *in vitro* in RPMI / 2mM Glutamin / 10% FCS (low IgG) medium. 50 µl aliquots of RL cell suspension (4 x 10E06 cell/ml) were incubated with immunoconjugates (2 µg/ml in BD FACS buffer) for 30 min at 4°C. Following washing with BD FACS buffer cells were stained with Alexa488 labeled anti-mouse IgG H+L (Invitrogen 65E1-1; 10 µg/ml in BD FACS buffer) for 30 min at 4 °C. After washing and centrifugation MFI signals of stained cells were analyzed by BD Biosciences FACSCanto flow cytometer. Results are shown in Figure 4.

The results from the FACS based cellular binding studies shown in Figure 4 demonstrate that all immunoconjugates bind to CD19 expressing RL cells in a comparable fashion.

### Example 4C:

### Internalization studies of immunoconjugates comprising monoclonal anti-CD19 antibody coupled to EBV and/or FLU peptide into CD19 expressing human non-Hodgkin's lymphoma cells (RL cells) and human Burkitt's lymphoma cell line (Ramos cells)

For the evaluation of internalizing capacity of generated immunoconjugates in comparison to parental anti-CD 19 mAb a FACS based internalization assay with the CD19 expressing cell lines RL and Ramos was performed.

Briefly, RL and Ramos cell were cultivated in vitro in RPMI / 2mM Glutamin / 10% FCS (low IgG) medium. 50 µl aliquots of RL and Ramos cell suspension (4 x 10E06 cells/ml) were incubated with immunoconjugates or parental anti-CD19 mAbs (5µg/ml in RL cell culture medium) for 30 min at 4°C. Following 2x washing with cold RL cell medium the cells were incubated in 100 µl medium at 4°C or 37°C for 30, 60, 120, 240 and 360 min and overnight [23h].

After the mentioned time points cells were washed, centrifuged and stained with Alexa488 labeled anti-human IgG H+L (JacksonImmuno 715-116-150 Lot 90812; 2,5µg/ml in BD FACS buffer) for 30 min at 4°C. After 2x washing and centrifugation cells were held in 100 µl BD Cell-Fix buffer and MFI signals of stained cells were analyzed by BD Biosciences FACSCanto flow cytometer.

No internalization was detected upon incubation at 4 °C. The results for the internalization studies at 37 °C are shown in Table 3.

**Table 3: Internalization of immunoconjugates comprising monoclonal anti-CD19 antibody coupled to EBV and/or FLU peptide into CD19 expressing human tumor cells (RL) at 37 °C**

| | **[%] Internalization into RL at 37 °C after** | | | | |
|---|---|---|---|---|---|
| | **30 min** | **60 min** | **240 min** | **360 min** | **23 h** |
| anti-CD19 MAb (positive control) | 43 | 57 | 80 | 84 | 90 |
| <CD19-SS-pEBV> | 46 | 65 | 81 | 85 | 92 |
| <CD19-SS-pEBV/FLU> | 43 | 63 | 82 | 92 | n.d. |
| anti-human IgG (negative control) | 0 | 0 | 0 | n.d. | 0 |

Results from the FACS based internalization studies show that the tested immunoconjugates internalize into CD19 expressing RL cells comparable to the parental anti-CD 19 reference antibody.

### Example 5:

### Activation of CD8 T cells after treatment of tumor cells with immunoconjugates comprising monoclonal anti-CDCPl antibody and EBV peptides and immunoconjugates comprising monoclonal anti-CD19 antibody and EBV peptides

### Example 5A:

### Generation of EBV peptide specific T cells from peripheral blood of healthy donors

The frequency of EBV peptide specific CD8 T cells in fresh isolated PBMCs from healthy donors or in T cell cultures was investigated in FACS via pentamer staining.

Briefly, aliquots of 4-5x10E05 T cell cultures or freshly isolated PBMCs from HLA-A2 matched healthy donors were washed with BD FACS buffer and incubated with 100 µL BD FACS buffer comprising 5 µL human TruStain FcX (BioLegend, 422302) for 15 min at room temperature. Afterwards 90 µL BD FACS buffer comprising 10µL of biotin conjugated EBV-Pentamer (ProImmune, F042-84A-E) was added and incubated for 1h at 4°C.

Upon washing with BD FACS buffer cells were stained in 100 µl BD FACS buffer with FITC-labelled anti-CD8a mAb (clone LT8, ProImmune) as well as streptavidin-APC conjugate at 4°C for 20 min. After 3x washing with BD FACS buffer and centrifugation cells were resuspended in 200 µl BD FACS buffer and MFI signals of stained cells were analyzed by BD Biosciences FACSCanto II flow cytometer. Cells from HLA-A1 donors were used for assessment of unspecific binding of tetramers.

EBV-peptide specific T cells were generated according to the protocol described in example 1 for the CMV derived peptide, with the difference that in this experiment the EBV peptide according to SEQ ID NO: 2 was used for stimulation.

### Example 5B:

### Evaluation of EBV peptide specific T cell activation after treatment of HCT-116 tumor cells with immunoconjugates comprising monoclonal anti-CDCPl antibody and EBV peptides and immunoconjugates comprising monoclonal anti-CD19 antibody and EBV peptides by IFNγ-ELISPOT

EBV peptide specific T cell activation was evaluated by IFNγ-ELISPOT using HCT-116 tumor cells treated with immunoconjugates (IC) from examples 3A, 3B (IC comprising anti-CDCPl and EBV peptides) and 4A (IC comprising anti-CD19 and EBV peptides) in presence of CD8 T cells obtained in example 5A. For comparative analyses the experiments were simultaneously carried out with free peptide (pEBV, positive control) and with a control immunoconjugate built up of an anti-Dig antibody coupled to the EBV peptide as described in example 3A (referred to as <DIG-SS-pEBV>, negative control).

The IFNγ-ELISPOT was performed according to the general protocol described in example 1B.

Results are shown in Fig. 5A.

The results from the IFNγ-ELISPOT demonstrate that from the tested immunoconjugates only those comprising monoclonal anti-CDCPl antibody and EBV peptides coupled via a cleavable bond (<CDCP1-SS-EBV>) sufficiently activate CD8 T cells upon internalization in tumor cells, whereas immunoconjugates comprising monoclonal anti-CDCPl antibody and EBV peptides coupled via a non-cleavable bond (<CDCP1-S-EBV>) were not capable of activating peptide-specific CD8 T cells. Immunoconjugates comprising antibodies that do not bind to the target cells (<DIG-SS-EBV>and <CD19-SS-EBV>) do not activate T-cells. Further, immunoconjugates according to the invention are capable of activating CD8 T cells in low amounts of even less than 1 nmol/l.

### Examnle 5C:

### Evaluation of EBV peptide specific T cell activation after treatment of RL and Ramos cells with immunoconjugates comprising monoclonal anti-CD19 antibody and EBV peptides by IFNγ-ELISPOT

In order to demonstrate that tumor cell killing is restricted to target cells expressing the HLA-subtype capable of presenting the respective T cell response eliciting peptide, EBV peptide specific T cell activation was evaluated by IFNγ-ELISPOT using RL cells, which express HLA-A2, and Ramos cells, which do not express HLA-A2, treated with immunoconjugates (IC) from example 4A (IC comprising anti-CD19 and HLA-A2 restricted EBV peptide) in presence of CD8 T cells obtained in example 5A. For comparative analyses the experiments were simultaneously carried out with free peptide (pEBV, positive control).

The IFNγ-ELISPOT was performed according to the general protocol described in example 1B.

Results are shown in Fig. 5B indicating that tumor cell killing was only observed in RL cells but not in Ramos cells.

### Example 6:

### Provision of immunoconjugate comprising a monoclonal anti-CDCPl antibody coupled to an EBV-peptide via a cleavable disulfide bond, wherein the EBV-peptide naturally comprises a non-terminal cysteine residue and assessment of CD8 T cell activation by IFNγ-ELISPOT

While in examples 3 and 4 immunoconjugates were provided, in which the T cell response eliciting peptides were *terminally* linked to the respective antibodies to form the immunoconjugate, in this example, the cleavable disulfide bond was formed between the *non-terminal* cysteine residue of the peptide and the antibody. For this, an EBV-peptide of the amino acid sequence GLCTLVAML (SEQ ID NO: 4) was provided and coupled to the anti-CDCPl antibody as described in example 3A.

Peptide specific T cell activation was evaluated by IFNγ-ELISPOT on different tumor cell lines expressing different levels of MHC I:
- MDA-MB 231, HLA-A2 high, and
- A375, HLA-A2 low.

In brief, 10,000 tumor cells were respectively treated with different concentrations of the immunoconjugate in presence of 500 peptide-specific CD8 T cells obtained as described in example 5B, however using the EBV-peptide GLCTLVAML instead. As controls, tumor cells and T cells were incubated in absence of immunoconjugate and, as a further control, T cells were incubated alone, under the same conditions.

Produced immunoconjugates according to the invention are herein referred to as follows:
- <CDCP1-SS-pEBV(GLC)> immunoconjugate comprising anti-CDCPl MAb coupled to EBV peptide GLCTLVAML by a disulfide bond

Results of the IFNγ-ELISPOT are shown in Fig. 5C. Peptide specific T cell activation was observed for both tested tumor cell lines for the immunoconjugate according to the invention in a dose dependent manner.

### Example 7:

### Mediation of CD8 T cell induced cell death in MDA-MB231 tumor cells treated with immunoconjugates according to the invention comprising monoclonal anti-CDCPl antibody and EBV peptides assessed by LDH release assay and xCELLigence

EBV peptide specific T cell activation and tumor cell killing was evaluated by an LDH release assay and by an xCELLigence assay using MDA-MB231 tumor cells treated with different concentrations of immunoconjugates (IC) from examples 3A, 3B (IC comprising anti-CDCPl and EBV peptides) in presence of CD8 T cells obtained in example 5A at an effector cell to target cell ratio of 3:1. For comparative analyses the experiments were simultaneously carried out with free peptide (pEBV, positive control).

The assessment of killing of tumor cells loaded with viral peptide as a result of treatment with immunoconjugates was performed via dynamic monitoring of cell proliferation and viability in xCELLigence System using the following general method: 50µL tumor cell medium was added per well and an initial background measurement (Step 1) at the RTCA instrument (Roche Applied Sciences) was performed. Substances suspended in 50µl tumor cell medium were added into E-plate. Tumor cells were harvested using Accutase and the respective cell numbers were suspended in 50µL tumor cell medium and added medium into E-plate.

The following cell numbers were used for the different tumor cell lines: A-375: 10.000 cells; HCT-116: 20.000 cells; PC-3: 10.000 cells; MDA-MB231: 10.000 cells.

After 10 min at room temperature the plates were placed in RTCA instrument and Step 2 was performed (Interval 10min, 200 Intervals). Subsequently, plates were incubated for about 24 hours.

On the next day, T cells were washed in AIM-V medium and suspended in AIM-V medium. T cells were added to the plates to a total volume of 200µl. Plates were placed into the RTCA instrument to perform Step 3 (Interval 5min, 300 Intervals). Plates were incubated overnight.

The samples were used for subsequent LDH release analysis.

For LDH release assessment, cells were lysed using adding 1% Triton X-100. Supernatant was transferred to a V-bottom 96-well plate and cell debris was removed by centrifugation and another transfer of the cell debris free supernatant to a flat bottom 96-well plate. LDH release was analysed using LDH cytotoxicity assay (Roche, #11644793001) according to the manufacturer's instructions. Briefly, catalyst and dye solution were mixed and 100µl of the resulting reaction mixture was added into each well and incubated at room temperature in the dark. Absorbance at 492nm (reference: 620nm) was measured using an Tecan ELISA reader at t=15-45 min (usually 30 min).

Results are shown in Fig. 6A and indicate that using immunoconjugates according to the invention, a potent tumor cell killing can be observed at picomolar concentrations.

### Example 8:

### Mediation of CD8 T cell induced cell death in MDA-MB231 and A-375 tumor cells treated with immunoconjugates according to the invention comprising monoclonal anti-CDCPl antibody and an EBV peptide, which naturally comprises a non-terminal cysteine residue, assessed by LDH release assay and xCELLigence

EBV peptide specific T cell activation and tumor cell killing was evaluated by an LDH release assay and by an xCELLigence assay and LDH release assessment using MDA-MB231 and A-375 tumor cells treated with different concentrations of the immunoconjugate (IC) of example 6 (IC comprising anti-CDCP1 and an EBV peptide, which naturally comprises a non-terminal cysteine residue) in presence of peptide specific CD8 T cells obtained in accordance with example 5A, however using the EBV-peptide GLCTLVAML instead for stimulation, at an effector cell to target cell ratio of 3:1. For comparative analyses the experiments were simultaneously carried out with free peptide (pEBV) in two different peptide concentrations.

The xCELLigence assay and LDH release assessment were performed as described in example 7.

Results are shown in Fig. 6B (for A-375 cells) and 6C (for MDA-MB231 cells) with the data of the xCELLigence assay being measured after 13,5 hours.

The results indicate that only the immunoconjugate according to the invention was capable of mediating the specific killing of the tested tumor cells via peptide specific T cells. In contrast, no specific tumor cell killing was observed using the equimolar concentration of free EBV peptide.

### Example 9:

### Mediation of CD8 T cell induced cell death in tumor cells treated with immunoconjugates according to the invention comprising monoclonal anti-CDCP1 antibody and FLU peptides assessed by LDH release assay and xCELLigence

Tumor cell killing of immunoconjugates according to the invention was assessed using HLA-A1 restricted FLU-peptide containing immunoconjugates (CDCP1-SS-pFLU, as obtained in example 3A) for treatment of different tumor cell lines (A-375, PC-3, HCT-116) in the presence of FLU-specific CD8 T cells. Peptide specific T cell activation and tumor cell killing was evaluated by an LDH release assay and by an xCELLigence assay as described in example 7.

The tested cell lines provide the following characteristics with respect to HLA-subtype and expression and expression of target (CDCP-1):

| **Cell line** | **Tumor type** | **HLA-A Subtype** | **CDCP-1 (MFI)** | **HLA-A1, A11, A26 / A1, A36 (MFI)** |
|---|---|---|---|---|
| PC-3 | Prostate | HLA-A1 | 43488 | 24452/1336 |
| HCT-116 | Intestine | HLA-A 1, A2 | 47678 | 24679/1747 |
| A-375 | Skin | HLA-A1, A2 | 1736 | 21758/710 |

Results from the xCELLigence analyses are shown in Figure 7A-C. Results from the LDH release assay are shown in Figure 7D-F.

Effective tumor cell killing upon treatment of tumor cells with the immunoconjugate was observed for all tested tumor cell lines. When compared to using free peptide, the immunoconjugate mediates T cell activation more efficiently than free peptide at a 100 fold higher concentration.

Tumor cell killing mediated by the immunoconjugate can be observed already 6 hours post treatment with the immunoconjugate indicating that the peptide does not require transportation to the antigen processing machinery in the cytosol for presentation via MHC class I. In addition, an increase in tumor cell killing over the course of time was observed, while - in contrast - tumor cell killing mediated by free peptide usually decreased in higher incubation times. Similar effects were observed in the different tumor cell lines, indicating that the effect is independent of the level of MHC class I expression in the target cell. The results of the xCELLigence analyses were confirmed by the results of the LDH release assay.

### Example 10: In vivo proof of concept I

### Anti-tumor activity of immunoconjugates according to the invention comprising monoclonal anti-CDCP1 antibody and EBV peptide in MDA-MB231 s.c. model with i.v. transfer of huPBMCs (comprising EBV-specific CD8 T cells)

NOG mice were xenografted with human tumor cells (5x10⁶ Mio MDA-MB231) via subcutanoues injection at day 0. Human PBMC stimulation (∼10% CLG-peptide EBV-specific CD8 T cells) was applied two times at day 0 and day 20 via i.v. transfer10x10⁶ (see detail in Figure 8A). Treament schedule of antibodies/ immunoconjugates was as follows:.

| | |
|---|---|
| <CDCP1>-IgG4-SS-CLG Peptide (generation as described in Example 3, with an IgG4 constant chain): | |
| | 5 mg/kg (on day: 0, 3, 6, 9, 13, 16, 20, 23, 27, 30) |
| Control AB: <CDCP1>-IgG4 (IgG4 constant chain): | |
| | 5 mg/kg (on day: 0, 3, 6, 9, 13, 16, 20, 23, 27, 30) |

Testing scheme and results are shown in Figure 8A and B. The results demonstrate the *in vivo* efficacy of immunoconjugates according to the invention comprising monoclonal anti-CDCPl antibody in MDA-MB231 s.c. xenograft model with adoptive transfer of CLG-specific huPBMCs.

### Example 11: In vivo proof of concent II

### Anti-tumor activity of immunoconjugates according to the invention comprising monoclonal anti-CDCPl antibody and EBV peptide in combination with anti-PDl treatment in established MDA-MB231 s.c. model with i.v. transfer of huPBMCs (comprising EBV-specific CD8 T cells)

NOG mice were xenografted with human tumor cells (5x10⁶ Mio MDA-MB231) via subcutanoues injection at day 0. Human PBMC stimulation (∼10% CLG-peptide EBV-specific CD8 T cells) was applied two times at day 20 and day 32 via i.v. transfer10x10⁶(see detail in Figure 8A). Treament schedule of the test group with test conjugate <CDCP1>-IgG1-PGLala-SS-CLG Peptide (generation as described in Example 3, with an IgG1 constant chain with mutations L234A, L235A and P329G (Kabat EU index) in the Fc part) and of the control group with the control antibody <CDCP1>-IgG1 PGLALA plus the addition of anti-PDl antibody for both groups was as follows:.

| | ***1st*** | ***2nd*** | ***3rd*** | ***4th*** | ***5th*** | ***6th*** | ***7th*** | ***8th*** | ***9th*** |
|---|---|---|---|---|---|---|---|---|---|
| **Treatment 1 (<CDCP1>- IgG1 PG LALA-SS- CLG Peptide /or control antibody <CDCP1>- IgG1 PG LALA, mg/kg)** | *2,5* | *2,5* | *5* | *5* | *10* | *10* | *15* | *15* | *20* |
| **Treatment 2 (PD1, mg/kg)** | | *5* | | | *5* | | | *5* | |

Testing scheme and results are shown in Figure 9A and B. The results demonstrate the combination effect of immunoconjugates according to the invention comprising monoclonal anti-CDCPl antibody plus anti-PD-1 therapy in MDA-MB231 xenograft model with adoptive transferred EBV CLG-specific huPBMC. A strong increase of CLG-specific T-cells in tumors after treatment with ATPP in combination with anti PD-1 antibody was shown.

## Claims

1. An immunoconjugate comprising at least one naturally occurring T cell response eliciting peptide that is presentable via MHC class I coupled to a target cell binding antibody via a cleavable bond, wherein the cleavable bond is arranged such that upon cleavage said T cell response eliciting peptide, which is directly presentable via a MHC class I molecule, is released from the immunoconjugate, **characterized in that** the naturally occurring T cell response eliciting peptide comprises at least one cysteine residue and **in that** the unmodified T cell response eliciting peptide is covalently coupled to the target cell binding antibody via a disulfide bond.

2. The immunoconjugate according to claim 1, wherein the target cell is a tumor cell.

3. The immunoconjugate according to any one of the preceding claims capable of being internalized into the target cell.

4. The immunoconjugate according to any one of the preceding claims, wherein the immunoconjugate does not comprise a translocation domain.

5. The immunoconjugate according to any one of the preceding claims, wherein in less than 12 hours after internalization of the immunoconjugate into the target cell the T cell response eliciting peptide is presented on the surface of the target cell via an MHC class I molecule.

6. A pharmaceutical composition comprising an immunoconjugate according to any one of the preceding claims in combination with a pharmaceutically acceptable carrier.

7. Use of a T cell response eliciting peptide that is presentable via MHC class I for the generation of an immunoconjugate according to any one of claims 1 to 5.

8. A method for the generation of an immunoconjugate according to any one of claims 1 to 5, comprising the steps of
a) providing the recombinant target cell binding antibody,
b) providing the T cell response eliciting peptide,
c) coupling of at least one of said T cell response eliciting peptide to said target cell binding antibody via the cleavable bond.

9. The method according to claim 8, wherein an unmodified T cell response eliciting peptide is coupled to the target cell binding antibody.

10. The immunoconjugate according to one of claims 1 to 5 for use as a medicament.

## Patentansprüche

1. Immunkonjugat, umfassend mindestens ein natürlich vorkommendes T-Zellantwort-auslösendes Peptid, das über MHC-Klasse I präsentierbar ist und das über eine spaltbare Bindung an einen Zielzell-bindenden Antikörper gebunden ist, wobei die spaltbare Bindung derart angeordnet ist, dass das T-Zellantwort-auslösende Peptid, das direkt über ein MHC-Klasse-I-Molekül präsentierbar ist, nach der Spaltung aus dem Immunkonjugat freigesetzt wird, **dadurch gekennzeichnet, dass** das natürlich vorkommende T-Zellantwort-auslösende Peptid mindestens einen Cysteinrest umfasst und dass das nicht modifizierte T-Zellantwort-auslösende Peptid über eine Disulfidbindung kovalent an den Zielzell-bindenden Antikörper gebunden ist.

2. Immunkonjugat nach Anspruch 1, wobei die Zielzelle eine Tumorzelle ist.

3. Immunkonjugat nach einem der vorhergehenden Ansprüche, das in der Lage ist, in die Zielzelle internalisiert zu werden.

4. Immunkonjugat nach einem der vorhergehenden Ansprüche, wobei das Immunkonjugat keine Translokationsdomäne umfasst.

5. Immunkonjugat nach einem der vorhergehenden Ansprüche, wobei das T-Zellantwort-auslösende Peptid in weniger als 12 Stunden nach der Internalisierung des Immunkonjugats in die Zielzelle über ein MHC-Klasse-I-Molekül an der Oberfläche der Zielzelle präsentiert wird.

6. Pharmazeutische Zusammensetzung, umfassend ein Immunkonjugat nach einem der vorhergehenden Ansprüche in Kombination mit einem pharmazeutisch unbedenklichen Träger.

7. Verwendung eines T-Zellantwort-auslösenden Peptids, das über MHC-Klasse I präsentierbar ist, für die Erzeugung eines Immunkonjugats nach einem der Ansprüche 1 bis 5.

8. Verfahren zur Erzeugung eines Immunkonjugats nach einem der Ansprüche 1 bis 5, umfassend die folgenden Schritte
a) Bereitstellen des rekombinanten Zielzell-bindenden Antikörpers,
b) Bereitstellen des T-Zellantwort-auslösenden Peptids,
c) Binden mindestens eines der T-Zellantwort-auslösenden Peptide an den Zielzell-bindenden Antikörper über die spaltbare Bindung.

9. Verfahren nach Anspruch 8, wobei ein nicht modifiziertes T-Zellantwort-auslösendes Peptid an den Zielzell-bindenden Antikörper gebunden wird.

10. Immunkonjugat nach einem der Ansprüche 1 bis 5 zur Verwendung als ein Medikament.

## Revendications

1. Immunoconjugué comprenant au moins un peptide provoquant une réponse des lymphocytes T existant à l'état naturel qui est présentable par l'intermédiaire du CMH de la classe I couplé à un anticorps se liant à la cellule cible par l'intermédiaire d'une liaison clivable, dans lequel la liaison clivable est agencée de sorte que, après le clivage, ledit peptide provoquant la réponse des lymphocytes T, qui est directement présentable par l'intermédiaire d'une molécule du CMH de la classe I, est libéré de l'immunoconjugué, **caractérisé en ce que** le peptide provoquant la réponse des lymphocytes T existant à l'état naturel comprend au moins un résidu cystéine et **en ce que** le peptide provoquant la réponse des lymphocytes T non modifié est couplé de manière covalente à l'anticorps se liant à la cellule cible par l'intermédiaire d'une liaison disulfure.

2. Immunoconjugué selon la revendication 1, dans lequel la cellule cible est une cellule tumorale.

3. Immunoconjugué selon l'une quelconque des revendications précédentes, pouvant être internalisé dans la cellule cible.

4. Immunoconjugué selon l'une quelconque des revendications précédentes, l'immunoconjugué ne comprenant pas un domaine de translocation.

5. Immunoconjugué selon l'une quelconque des revendications précédentes, dans lequel, moins de 12 heures après l'internalisation de l'immunoconjugué dans la cellule cible, le peptide provoquant la réponse des lymphocytes T est présenté sur la surface de la cellule cible par l'intermédiaire d'une molécule du CMH de la classe I.

6. Composition pharmaceutique comprenant un immunoconjugué selon l'une quelconque des revendications précédentes en combinaison avec un véhicule pharmaceutiquement acceptable.

7. Utilisation d'un peptide provoquant la réponse des lymphocytes T qui est présentable par l'intermédiaire du CMH de la classe I pour la génération d'un immunoconjugué selon l'une quelconque des revendications 1 à 5.

8. Procédé pour la génération d'un immunoconjugué selon l'une quelconque des revendications 1 à 5, comprenant les étapes de :
a) fourniture de l'anticorps recombinant se liant à la cellule cible,
b) fourniture du peptide provoquant la réponse des lymphocytes T,
c) couplage d'au moins un dudit peptide provoquant la réponse des lymphocytes T audit anticorps se liant à la cellule cible par l'intermédiaire de la liaison clivable.

9. Procédé selon la revendication 8, dans lequel un peptide provoquant la réponse des lymphocytes T non modifié est couplé à l'anticorps se liant à la cellule cible.

10. Immunoconjugué selon l'une des revendications 1 à 5 pour une utilisation comme médicament.


### SEQUENCE LISTING

<110> F. Hoffmann-La Roche AG
<120> IMMUNOCONJUGATES FOR SPECIFIC INDUCTION OF T CELL CYTOTOXICITY AGAINST A TARGET CELL
<130> P32622-WO
<150> EP15155598
   <151> 2015-02-18
<150> EP15199306
   <151> 2015-12-10
<160> 10
<170> PatentIn version 3.5
<210> 1
   <211> 9
   <212> PRT
   <213> Human herpesvirus 5
<220>
   <221> misc_feature
   <223> Human herpesvirus 5 (CMV) peptide pp65 495-503
<400> 1
   Asn Leu Val Pro Met Val Ala Thr Val
   1               5
<210> 2
   <211> 9
   <212> PRT
   <213> Epstein-Barr virus
<220>
   <221> misc_feature
   <223> Epstein-Barr virus (EBV) peptide LMP-2 426-434
<400> 2
   Cys Leu Gly Gly Leu Leu Thr Met Val
   1               5
<210> 3
   <211> 9
   <212> PRT
   <213> Influenza A virus
<220>
   <221> misc_feature
   <223> Influenza A (FLU) peptide NP 44-52
<400> 3
   Cys Thr Glu Leu Lys Leu Ser Asp Tyr
   1               5
<210> 4
   <211> 9
   <212> PRT
   <213> Epstein-Barr virus
<220>
   <221> misc_feature
   <223> Epstein-Barr virus (EBV) peptide BLMF-1 280-288
<400> 4
   Gly Leu Cys Thr Leu Val Ala Met Leu
   1               5
<210> 5
   <211> 9
   <212> PRT
   <213> Human herpesvirus 5
<220>
   <221> misc_feature
   <223> Human herpesvirus 5 (CMV) peptide 309-317
<400> 5
   Cys Arg Val Leu Cys Cys Tyr Val Leu
   1               5
<210> 6
   <211> 10
   <212> PRT
   <213> Artificial Sequence
<220>
   <223> modified Human herpesvirus 5 (CMV) peptide pp65 495-503 (cCMV peptide)
<400> 6
   Cys Asn Leu Val Pro Met Val Ala Thr Val
   1               5                   10
<210> 7
   <211> 10
   <212> PRT
   <213> Artificial Sequence
<220>
   <223> modified Human herpesvirus 5 (CMV) peptide pp65 495-503 (CMVc peptide)
<400> 7
   Asn Leu Val Pro Met Val Ala Thr Val Cys
   1               5                   10
<210> 8
   <211> 9
   <212> PRT
   <213> Artificial Sequence
<220>
   <223> modified Human herpesvirus 5 (CMV) peptide pp65 495-503 (rcCMV peptide)
<400> 8
   Cys Leu Val Pro Met Val Ala Thr Val
   1               5
<210> 9
   <211> 9
   <212> PRT
   <213> Artificial Sequence
<220>
   <223> modified Human herpesvirus 5 (CMV) peptide pp65 495-503 (CMVrc peptide)
<400> 9
   Asn Leu Val Pro Met Val Ala Thr Cys
   1               5
<210> 10
   <211> 9
   <212> PRT
   <213> Artificial Sequence
<220>
   <223> modified Human herpesvirus 5 (CMV) peptide pp65 495-503 (CMVpen peptide)
<220>
   <221> misc_feature
   <223> Xaa is penicillamine
<220>
   <221> misc_feature
   <222> (9)..(9)
   <223> Xaa can be any naturally occurring amino acid
<400> 10
   Asn Leu Val Pro Met Val Ala Thr Xaa
   1               5